# EUROPEAN PATENT APPLICATION

(11) **EP 3 412 688 A1**
(43) Date of publication of application: **12.12.2018**
(21) Application number: 17746993.9
(22) Date of filing: 03.02.2017
(51) Int. Cl.: C07K 16/40, C07K 16/46, A61K 39/395, A61P 7/02, A61P 9/10

(54) **THROMBIN ANTIBODY, ANTIGEN-BINDING FRAGMENT AND PHARMACEUTICAL USE THEREOF**

(30) Priority: 05.02.2016 CN 201610083070; 11.04.2016 CN 201610223995
(71) Applicant: Jiangsu Hengrui Medicine Co., Ltd., Jiangsu 222047 (CN); Shanghai Hengrui Pharmaceutical Co. Ltd., Minhang District Shanghai 200245 (CN)
(72) Inventor: YING, Hua, Shanghai 200245 (CN); LIU, Jiajian, Shanghai 200245 (CN); FU, Yayuan, Shanghai 200245 (CN); ZHANG, Ling, Shanghai 200245 (CN); ZHANG, Haoying, Shanghai 200245 (CN); SUN, Jiakang, Shanghai 200245 (CN); ZHANG, Lianshan, Shanghai 200245 (CN); TAO, Weikang, Shanghai 200245 (CN); SUN, Piaoyang, Lianyungang Jiangsu 222047 (CN); HU, Qiyue, Shanghai 200245 (CN)
(74) Representative: Gevers & Orès
(86) International application number: PCT/CN2017/072850
(87) International publication number: WO 2017/133673

(57) **Abstract**

Provided are a thrombin antibody, an antigen-binding fragment and a pharmaceutical use thereof. Further provided are a chimeric antibody and a humanized antibody comprising a thrombin antibody CDR region, and a pharmaceutical composition comprising the thrombin antibody and the antigen-binding fragment thereof, as well as the use thereof as an anticoagulant drug. In particular, provided is a use of the humanized thrombin antibody in preparing a drug for treating a thrombin-mediated disease or condition.

## Description

### FIELD OF THE INVENTION

The present invention relates to a thrombin antibody, and antigen-binding fragments thereof. Further, the present invention relates to a chimeric antibody, a humanized antibody comprising CDR regions of the thrombin antibody. The present invention further relates to a pharmaceutical composition comprising the thrombin antibody and antigen-binding fragments thereof, as well as use of the antibody as anticoagulant agent.

### BACKGROUND OF THE INVENTION

Blood coagulation is a key process in preventing damaged blood vessels from bleeding (hemostasis). However, a blood clot that blocks the blood to flow through a vessel (thrombosis) or a shed blood clot that deposits in a blood vessel elsewhere in the body (thromboembolism) can be a serious threat to health. Thrombosis (such as acute myocardial infarction (AMI), venous thromboembolism, etc.) is a serious cardiovascular disease that hazards human health and life. According to the statistics from the World Health Organization in 2008, cardiovascular disease incidence and mortality have ranked first. Every year about 17.33 million people die from cardiovascular diseases in the world, accounting for 30% of the total mortality. There are 290 million cardiovascular patients in China, and about 3.5 million people die every year, accounting for 41% of total mortality. According to the statistics from Global Disease Burden Study (GBD) in 2010, stroke is the leading cause of death for residents in our country. Therefore, researches on effective drugs and methods for treatment of cardiovascular diseases have attracted more and more attention in recent years. Currently, a few anticoagulant therapies can be used to treat pathological blood coagulation, such as traditional drug heparin, small molecule heparin or warfarin, or direct use of thrombin inhibitor, dabigatran (Dabigatran), etc. The general disadvantage of these therapies is the increased risk of bleeding. With respect to many anticoagulants, the window between the effective dose (preventing from thrombosis) and the safe dose (the highest dose without risk of bleeding) is not wide enough, and the window will even be narrowed in light of the individual difference in response. Use of thrombin antagonists, which are targeting thrombin, to inhibit the formation of thrombus, is one of the methods for clinical treatment of thrombosis.

Coagulation reaction is a complex signal cascade process, and thrombin plays a central role in this process. Thrombin breaks down the fibrinogen in the circulatory system into fibrin monomers (which can be aggregated to form fibrin, a fiber matrix of the blood clots), and it also directly controls cells. As a serine protease, it triggers platelets to be deformed, and release ADP, a platelet activator, serotonin and thromboxane A2, as well as chemokines and growth factors. In addition, it also promotes the migration of adhesion molecule P-selection and CD 40 ligand to the surface of platelets, and then activates integrin allb/b3. The latter combines with fibrinogen and von Willebrand factor (vWF), and then mediates platelet aggregation. Thrombin also stimulates the procoagulant activity of the platelet surface, which in turn promotes the expression of thrombin. In the cultured endothelial cells, thrombin promotes the release of vWF, the appearance of P-selectin in plasma membrane and the production of chemokines. These reactions are believed to trigger the binding of platelets and leukocytes to the surface of endothelial cells. Endothelial cells immediately change their shapes and the permeability of endothelial cell layer is increased. These reactions are expected to promote local exudation of plasma proteins and promote edema. In non-endothelial tissue, thrombin induces vasoconstriction by acting on smooth muscle cells. In the cultured fibroblasts or vascular smooth muscle cells in vitro, thrombin regulates the production of cytokines and promotes mitosis. In T lymphocytes, it triggers calcium signals and other reactions. These cellular responses indicate that thrombin correlates tissue damage with hemostatic process, inflammatory response, and even the regulation of body that enhances the immune response. These cell responses also suggest a possibility that, in addition to tissue damage, thrombin in endothelial cells and other types of cells may also play a role in leukocytic exocytosis, vascular remodeling and/or angiogenesis. Therefore, thrombin has become a potential anticoagulant and anti-thrombotic target.

The isolated anti-thrombin antibody can inhibit thrombin in vivo, without promoting or significantly promoting bleeding (bleeding) or haemorrhage, that is, the antibody molecule does not inhibit or substantially inhibit the normal physiological response to vascular injury (hemostasis). For example, hemostasis will not be suppressed by the antibody molecule or will be minimally suppressed (i.e., slightly inhibited, without affecting the patient's health or no requiring further intervention). Bleeding will not be increased by antibody molecules or be minimally increased.

Although these is a few patent disclosures with respect to anti-thrombin antibodies, such as WO2013123591, WO2014153195, WO2014202992 and WO2014202993, there is not any anti-thrombin antibody available as an approved medicament or entering into clinical research so far. It is necessary to further develop a new anti-thrombin antibody for the relating clinical research and application. The present invention provides a novel thrombin antibody with high affinity and remarkable inhibitory activity against thrombosis.

### SUMMARY OF THE INVENTION

The present invention provides a thrombin antibody or antigen-binding fragment thereof, comprising one or more CDR region selected from the following sequence or an amino acid sequence having at least 95% identity to the following sequence:
HCDR region of antibody heavy chain variable region: SEQ ID NO: 7, SEQ ID NO:8 and SEQ ID NO: 21; and
LCDR region of antibody light chain variable region: SEQ ID NO:10, SEQ ID NO:11 and SEQ ID NO: 12;
wherein the sequence of SEQ ID NO: 21 is represented by formula (I):

   DHYX₁G X₂SYVFD X₃ (I);

   wherein X₁ is selected from the group consisting of H, I, L and M; X₂ is selected from N and A; X₃ is selected from the group consisting of Y, S, L and T.

In another preferred embodiment of the thrombin antibody or the antigen-binding fragment thereof of the present invention, wherein the thrombin antibody or the antigen-binding fragment thereof comprises a HCDR1, a HCDR2, and a HCDR3 as shown in SEQ ID NO:7, SEQ ID NO:8, and SEQ ID NO: 21, respectively, or an amino acid sequence having at least 95% identity to SEQ ID NO:7, SEQ ID NO:8, and SEQ ID NO: 21.

In another preferred embodiment of the thrombin antibody or the antigen-binding fragment thereof of the present invention, wherein the sequence SEQ ID NO: 21 of formula (I) is selected from the following sequence:
DHYHGNSYVFDY SEQ ID NO: 9;
DHYIGASYVFDY SEQ ID NO: 23;
DHYLGNSYVFDS SEQ ID NO: 24;
DHYLGNSYVFDL SEQ ID NO: 25; or
DHYMGNSYVFDT SEQ ID NO: 26.

In another preferred embodiment of the thrombin antibody or the antigen-binding fragment thereof of the present invention, wherein the thrombin antibody or the antigen-binding fragment thereof comprises a LCDR1, a LCDR2, and a LCDR3 as shown in SEQ ID NO: 10, SEQ ID NO: 11, and SEQ ID NO: 12, respectively, or an amino acid sequence having at least 95% identity to SEQ ID NO: 10, SEQ ID NO: 11, and SEQ ID NO: 12.

In another preferred embodiment of the thrombin antibody or the antigen-binding fragment thereof of the present invention, wherein the thrombin antibody or the antigen-binding fragment thereof is a murine antibody or the functional fragment thereof.

In another preferred embodiment of the thrombin antibody or the antigen-binding fragment thereof of the present invention, wherein the thrombin antibody or the antigen-binding fragment thereof further comprises a light chain FR region derived from murine κ chain or a variant thereof, or further comprises a light chain FR region derived from murine λ chain or a variant thereof; and/or wherein the thrombin antibody or the antigen-binding fragment thereof further comprises a heavy chain FR region derived from murine IgG1 or a variant thereof, or further comprises a heavy chain FR region derived from murine IgG2 or a variant thereof, or further comprises a heavy chain FR region derived from murine IgG3 or a variant thereof.

In another preferred embodiment of the the thrombin antibody or the antigen-binding fragment thereof of the present invention, wherein the thrombin antibody or the antigen-binding fragment thereof comprises a heavy chain variable region of SEQ ID NO: 5 and a light chain variable region of SEQ ID NO: 6.

In another preferred embodiment of the the thrombin antibody or the antigen-binding fragment thereof of the present invention, wherein the thrombin antibody or the antigen-binding fragments thereof further comprises a light chain constant region derived from murine κ chain or a variant thereof, or a light chain constant region derived from murine λ chain or a variant thereof; and/or wherein the thrombin antibody or the antigen-binding fragment thereof further comprises a heavy chain FR region derived from murine IgG1 or a variant thereof, or a heavy chain FR region derived from murine IgG2 or a variant thereof, or a heavy chain FR region derived from murine IgG3 or a variant thereof.

In another preferred embodiment of the thrombin antibody or the antigen-binding fragment thereof of the present invention, wherein the thrombin antibody or the antigen-binding fragment thereof is a chimeric antibody or the functional fragment thereof.

In another preferred embodiment of the thrombin antibody or the antigen-binding fragment thereof of the present invention, wherein the thrombin antibody or the antigen-binding fragments thereof is a humanized antibody or the functional fragment thereof.

In another preferred embodiment of the thrombin antibody or the antigen-binding fragment thereof of the present invention, wherein the heavy chain FR region of humanized antibody or the functional fragment thereof is derived from a combination sequence of human germline heavy chains IGHV3-23*04 and hjh6.1 or a mutant sequence thereof; wherein the humanized antibody or the functional fragment thereof comprises FR1, FR2, FR3 region from human germline heavy chain IGHV3-23*04 and FR4 region from human germline heavy chain hjh6.1 or a mutant sequence thereof.

In another preferred embodiment of the thrombin antibody or the antigen-binding fragment thereof of the present invention, wherein the humanized antibody or the functional fragment thereof comprises a heavy chain variable region of SEQ ID NO: 13, or an amino acid sequence having at least 95% identity to SEQ ID NO: 13; preferably an amino acid sequence having 0-10 amino acid changes in SEQ ID NO: 13.

In another preferred embodiment of the thrombin antibody or the antigen-binding fragment thereof of the present invention, wherein the humanized antibody or the functional fragment thereof comprises a heavy chain FR region with 0-10 amino acid back-mutations, wherein the back-mutation is preferably selected from the group consisting of R87K, K98R, Q3K and S49A amino acid back-mutations.

In another preferred embodiment of the thrombin antibody or the antigen-binding fragment thereof of the present invention, wherein the humanized antibody or the functional fragment thereof comprises a heavy chain variable region of SEQ ID NO: 22 or SEQ ID NO: 15, or an amino acid sequence having at least 95% identity to SEQ ID NO: 22 or SEQ ID NO: 15;
wherein the sequence of SEQ ID NO: 22 is represented by formula (II): EVQLVESGGGLVQPGGSLRLSCAASGFTFSDYYMAWVRQAPGKGLEW 
VSNINSDGSSTYYLDSLKSRFTISRDNSKNTLYLQMNSLKAEDTAVYYCARDH
YX1G X2SYVFDX3WGQGTTVTVSS (II);
 wherein X₁ is selected from the group consisting of H, I, L and M; X₂ is selected from N and A; X₃ is selected from the group consisting of Y, S, L and T.

In another preferred embodiment of the thrombin antibody or the antigen-binding fragment thereof of the present invention, wherein the sequence SEQ ID NO: 22 of formula (II) is selected from the group consisting of SEQ ID NO: 14, SEQ ID NO: 27, SEQ ID NO: 28, SEQ ID NO: 29 and SEQ ID NO: 30.

In another preferred embodiment of the thrombin antibody or the antigen-binding fragment thereof of the present invention, wherein the light chain FR region of humanized antibody or the functional fragments thereof is derived from a combination sequence of human germline light chains IGKV1-39*01 and hjk4.1 or a mutant sequence thereof; wherein the humanized antibody or the functional fragment thereof comprises FR1, FR2, FR3 region from human germline light chain IGKV1-39*01 and FR4 region from hjk4.1 or a mutant sequence thereof.

In another preferred embodiment of the thrombin antibody or the antigen-binding fragment thereof of the present invention, wherein the humanized antibody or the functional fragment thereof comprises a light chain variable region of SEQ ID NO: 16, or an amino acid sequence having at least 95% identity to SEQ ID NO: 16; preferably an amino acid sequence having 0-10 amino acid changes compared to SEQ ID NO: 16.

In another preferred embodiment of the thrombin antibody or the antigen-binding fragment thereof of the present invention, wherein the humanized antibody or the functional fragment thereof comprises light chain FR region with 0-10 amino acid back-mutations, wherein the back-mutation is preferably selected from the group consisting of Y49S, T69K, K45R, L47I and F71Y amino acid back-mutations.

In another preferred embodiment of the thrombin antibody or the antigen-binding fragment thereof of the present invention, wherein the humanized antibody or the functional fragment thereof comprises a light chain variable region of SEQ ID NO: 17 or SEQ ID NO: 18, or an amino acid sequence having at least 95% identity to SEQ ID NO: 17 or SEQ ID NO: 18.

In another preferred embodiment of the thrombin antibody or the antigen-binding fragment thereof of the present invention, wherein the humanized antibody comprises:
a) heavy chain variable region, wherein the sequence of the heavy chain variable region is selected from the group consisting of SEQ ID NO:13, SEQ ID NO:14, SEQ ID NO: 15, and an amino acid sequence having at least 95% identity to SEQ ID NO:13, SEQ ID NO:14, SEQ ID NO: 15; and/or
b) light chain variable region, wherein the sequence of the light chain variable region is selected from the group consisting of SEQ ID NO: 16, SEQ ID NO:17, SEQ ID NO: 18, and an amino acid sequence having at least 95% identity to SEQ ID NO:16, SEQ ID NO:17, SEQ ID NO: 18.

In another preferred embodiment of the thrombin antibody or the antigen-binding fragment thereof of the present invention, wherein the thrombin antibody or the antigen-binding fragments comprises a heavy chain constant region derived from human IgG1, IgG2, IgG3 or IgG4, or a variant thereof, preferably comprises a heavy chain constant region derived from human IgG4, most preferably comprises a constant region of SEQ ID NO: 19.

The thrombin antibody or the antigen-binding fragments further comprises a light chain constant region derived from human κ chain, human λ chain or a variant thereof, most preferably comprises a light chain constant region of SEQ ID NO: 20.

In another preferred embodiment of the thrombin antibody or the antigen-binding fragment thereof of the present invention, wherein the antigen-binding fragment is selected from the group consisting of Fab, Fab', F(ab')₂, single-chain antibody(scFv), dimerized V region (diabody), disulfide stabilized V region (dsFv) and antigen-binding fragment comprising CDR peptide.

In another preferred embodiment of the thrombin antibody or the antigen-binding fragment thereof of the present invention, wherein the thrombin antibody or the antigen-binding fragment binds to one or more residues selected from the group consisting of R70, Y71, E72, R73, N74, 175 and Y114 of the thrombin; preferably, the thrombin antibody or the antigen-binding fragment further binds to residues at positions 9, 24, 60, 62, 64, 66, 69, 78 and 113 of the thrombin.

The present invention further providers an isolated monoclonal antibody or antigen-binding fragments thereof that binds to one, two, three, four, five, six or seven residues selected from the group consisting of R70, Y71, E72, R73, N74, 175, and Y114 of the thrombin; preferably, the thrombin antibody or the antigen-binding fragments thereof further binds to residues at positions 9, 24, 60, 62, 64, 66, 69, 78 and 113 of the thrombin.

The present invention further providers a pharmaceutical composition, comprising therapeutically effective amount of the thrombin antibody or the antigen-binging fragment thereof as described above, and one or more pharmaceutically acceptable carriers, diluents or excipients.

The present invention further providers a DNA molecule encoding the thrombin antibody or antigen-binding fragment thereof as described above.

The present invention further provides an expression vector comprising the DNA molecule as described above.

The present invention further provides a host cell transformed with the expression vector as described above, wherein the host cell is selected from the group consisting of a prokaryotic cell and a eukaryotic cell, preferably a eukaryotic cell, more preferably a mammalian cell.

The present invention further provides use of the thrombin antibody or antigen-binding fragment thereof or the pharmaceutical composition as described above, in the preparation of a medicament for treatment of thrombin-mediated disease or condition, wherein the disease or condition is preferably a thrombotic disease including thrombosis and thromboembolism; more preferably, venous thrombosis and pulmonary embolism, arterial thrombosis, thrombosis-induced stroke and peripheral arterial formation, atherosclerotic disease, cerebral arterial disease, or peripheral arterial disease; most preferably venous thrombosis, thrombosis-induced stroke, and atherosclerosis.

The exemplary diseases which can be diagnosed with the antibody according to the present invention include thrombotic disorders (including thrombosis and thromboembolism), including any one or more disease selected from the group consisting of: venous thrombosis and pulmonary embolism, arterial thrombosis, thrombosis-induced stroke and peripheral arterial formation, atherosclerotic disease, cerebral arterial disease, or peripheral arterial disease.

In one aspect, the present invention provides a method of treating or preventing hypercholesterolemia and/or at least one symptom selected from venous thrombosis, thrombosis-induced stroke and atherosclerosis, wherein the method comprises administrating an effective amount of the anti-thrombin antibody to the individual. The present invention also provides use of an effective amount of thrombin antibodies against extracellular or circulating thrombin in the preparation of a medicament, wherein the medicament is used for treating or preventing thrombosis and/or at least one symptom selected from pulmonary embolism, thrombosis-induced stroke and atherosclerosis in an individual.

In one aspect, the present invention provides an agent for detecting or measuring human thrombin, wherein the agent comprising the antibody or antigen-binding fragment thereof as described above.

### BRIEF DESCRIPTION OF THE DRAWINGS

Figure 1: Effect of the anti-thrombin antibody CH-1601 on human thrombinase activity; Data showed that the binding of CH-1601 to thrombin did not affect the enzymatic activity of thrombin on substrate S2228.
Figure2: Effect of the anti-thrombin antibody of present invention on human thrombinase activity; Data showed that the binding of H1601-008 to thrombin did not affect the enzymatic activity of thrombin on substrate S2228.
Figure3: The effect of various concentrations of the thrombin antibodies on plasma APTT value in normal human; Data showed that with the increase of the concentrations of antibodies CH-1601 and h1601-008, the APTT value of normal human plasma is also increased; at the highest concentration of 3200 nM, for h1601-008, the APTT value was also increased to a peak of 28.7 seconds.
Figure4: Effect of intravenous injection of H1601-008 on venous thrombosis in monkey AV-shunt.
Figure5: Effect of the antibodies of the present invention on the APTT value of monkey plasma.
Figure6: Purified antibody H1601-008 is cleaved with papain.
Figure7: A diagram showing crystal structure of Fab, wherein LCDR1, LCDR2, LCDR3, HCDR3 are tightly bound to thrombin.
Figure8: Residues R62, Y71, E72, R73 and Y114 of thrombin have hydrogen-bond interactions with the four rings of the Fab, respectively.

### DETAILED DESCRIPTION OF THE INVENTION

### Terms

In order to more readily understand the invention, certain technical and scientific terms are specifically defined below. Unless specifically defined elsewhere in this document, all other technical and scientific terms used herein have the meaning commonly understood by one of ordinary skill in the art to which this invention belongs.

As used herein, the single-letter code and the three-letter code for amino acids are as described in J. Biol. Chem, 243, (1968) p3558.

As used herein, "antibody" refers to immunoglobulin, a four-peptide chain structure connected together by disulfide bonds between two identity heavy chains and two identity light chains. Different immunoglobulin heavy chain constant regions exhibit different amino acid compositions and rank orders, hence present different kinds of antigenicity. Accordingly, immunoglobulin can be divided into five categories, or called as immunoglobulin isotypes, namely IgM, IgD, IgG, IgA and IgE, their corresponding heavy chains are µ chain, δ chain, γ chain, α chain and ε chain, respectively. According to its amino acid composition of hinge region and the number and location of heavy chain disulfide bonds, the same type of Ig can be divided into different sub-categories, for example, IgG can be divided into IgG1, IgG2, IgG3, and IgG4. Light chain can be divided into κ or λ chain considering of different constant region. Each of the five Igs can have κ or λ chain.

In the present invention, the antibody light chain mentioned herein further comprises a light chain constant region, which comprises a human or murine κ, λ chain or a variant thereof.

In the present invention, the antibody heavy chain mentioned herein further comprises a heavy chain constant region, which comprises human or murine IgG1, IgG2, IgG3, IgG4 or a variant thereof.

Near the N-terminal sequence of the antibody heavy and light chains, about 110 of amino acids change largely, known as variable region (F_{V} region); the rest of the amino acid sequences near the C-terminus are relative stable, known as constant region. Variable region comprises three hypervariable regions (HVR) and four relatively conserved sequence framework regions (FR). Three hypervariable regions determine the specificity of the antibody, also known as complementarity determining region (CDR). Each light chain variable region (LCVR) and each heavy chain variable region (HCVR) is composed of three CDR regions and four FR regions, with sequential order from the amino terminus to the carboxyl terminus being: FR1, CDR1, FR2, CDR2, FR3, CDR3, and FR4. Three light chain CDRs refer to LCDR1, LCDR2, and LCDR3; three heavy chain CDRs refer to HCDR1, HCDR2 and HCDR3. The number and location of CDR region amino acid residues in LCVR and HCVR regions of the antibody or antigen binding fragment herein comply with the known Kabat numbering criteria (LCDR1-3, HCDE2-3), or comply with Kabat and Chothia numbering criteria (HCDR1).

The antibody of the present invention comprises murine antibody, chimeric antibody and humanized antibody, preferable humanized antibody.

The term "murine antibody" used in the present invention refers to anti-human thrombin monoclonal antibody prepared according to the knowledge and skills known in the field. During the preparation, a test subject was injected with thrombin antigen, and then hybridoma expressing antibody which possesses desired sequence or functional characteristics was separated. In a preferred embodiment of the present invention, the murine thrombin antibody or antigen binding fragment thereof, further comprises light chain constant region of murine κ, λ chain or a variant thereof, or further comprises heavy chain constant region of murine IgG1, IgG2, IgG3, or a variant thereof.

The term "chimeric antibody", is an antibody which is formed by fusing the variable region of a murine antibody with the constant region of a human antibody, the chimeric antibody can alleviate the murine antibody-induced immune response. To establish chimeric antibody, hybridoma secreting specific murine monoclonal antibody is first established, a variable region gene is cloned from murine hybridoma cells, then a constant region gene of a human antibody is cloned as desired, the murine variable region gene is ligated with human constant region gene to form a chimeric gene which can be inserted into an expression vector, and finally the chimeric antibody molecule is expressed in the eukaryotic or prokaryotic system. In a preferred embodiment of the present invention, the light chain of the thrombin chimeric antibody further comprises the light chain constant regions of human κ, λ chain or a variant thereof. The heavy chain of the thrombin chimeric antibody further comprises the heave chain constant regions of human IgG1, IgG2, IgG3, IgG4 or a variant thereof, preferably comprises heavy chain constant region of human IgG1, IgG2, IgG3 or IgG4, or preferably comprises heavy chain constant region of human IgG1, IgG2 or IgG4, or a variant thereof with amino acid mutations (e.g., YTE mutation).

The term "humanized antibody", also known as CDR-grafted antibody, refers to an antibody generated by grafting murine CDR sequences into a variable region framework of a human antibody, namely, an antibody produced among different types of human germline antibody framework sequences. Humanized antibody overcomes the heterologous response induced by the chimeric antibody which carries a large amount of murine protein components. Such framework sequences can be obtained from public DNA database covering germline antibody gene sequences or published references. For example, germline DNA sequences of human heavy and light chain variable region genes can be found in "VBase" human germline sequence database (available on web www.mrccpe.com.ac.uk/vbase), as well as can be found in Kabat, EA, et al, 1991 Sequences of Proteins of Immunological Interest, 5th Ed. To avoid the decrease in the activity during immunogenicity reduction, the variable region frame sequence of the human antibody is subjected to a minimum back mutation to maintain the activity. The humanized antibody of the present invention also comprises a humanized antibody which is further subjected to CDR affinity maturation by phage display. In a preferred embodiment of the present invention, the murine CDR sequences of the humanized thrombin antibody are selected from the group consisting of SEQ ID NOs: 7, 8, 9, 10, 11 and 12; The variable region frame of human antibody is designed and selected, wherein the heavy chain FR sequence of the heavy chain variable region of the antibody is derived from a combination sequence of human germline heavy chains IGKV1-39*01 and hjk4.1; wherein the light chain FR sequence of the light chain variable region of the antibody is derived from a combination sequence of human germline heavy chains IGHV3-23*04 and hjh6.1. In order to avoid the decrease of the activity caused by the decrease of immunogenicity, the variable region of human antibody described herein can be subjected to minimal back-mutations to maintain the activity of antibody.

The grafting of CDRs may result in a decrease in the affinity of the thrombin antibody or antigen-binding fragment thereof to the antigen due to the change of framework residues in contact with the antigen. Such interactions may be the result of highly somatic mutations. Thus, it may still be necessary to implant such donor framework amino acids to the framework of humanized antibodies. The amino acid residues involved in antigen binding of nonhuman thrombin antibody or antigen-binding fragment thereof can be identified by examining the variable region sequence and structure of murine monoclonal antibody. Each of the residues in the CDR donor framework that is different from the germline may be considered to be relevant. If it is not possible to determine the most closely related species, the sequence can be compared to a consensus sequence of a subtype consensus sequence or a murine sequence with a high similarity percentage. Rare frame residues are thought to be the result of a highly somatic cell mutation, which plays an important role in binding.

The term "antigen-binding fragment" of an antibody or "function fragment", refers to one or more fragments of an antibody that retain the ability to specifically bind to an antigen (*e*. *g*., a thrombin protein). It has been shown that the antigen-binding function of an antibody can be performed by fragment of a full length antibody. Examples of binding fragments encompassed within the term "antigen binding fragment" of an antibody include (i) a Fab fragment, a monovalent fragment consisting of the VL, VH, CL and CH1 domains; (ii) a F(ab')₂ fragment, a bivalent fragment comprising two Fab fragments linked by a disulfide bridge on the hinge region; (iii) a Fd fragment consisting of the VH and CH1 domains; (iv) a Fv fragment consisting of the VL and VH domains of a single arm of an antibody; (v) a single domain or a dAb fragment (Ward et al., (1989) Nature10 341:544-546), which consists of a VH domain; and (vi) a separated complementarity determining region (CDR) or (vii) optionally a combination of two or more separated CDRs linked by a synthetic linker. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); *see e.g.,* Bird et al. (1988) Science 242:423-426; and Huston et al. (1988) Proc. Natl. Acad. Sci. USA 85:5879-5883). Such single chain antibodies are also intended to be encompassed within the term "antigen binding fragment" of an antibody. These antibody fragments are obtained using conventional techniques known to those with skill in the art, and the functions of the fragments are screened in the same manner as the intact antibodies. The antigen-binding moiety can be produced by recombinant DNA techniques or by enzymatic or chemical cleavage of intact immunoglobulins. The antibodies may be antibodies of different isoforms, for example, IgG (e.g., IgG1, IgG2, IgG3 or IgG4 subtype), IgA1, IgA2, IgD, IgE or IgM antibodies.

The term "single chain antibody", "single chain Fv" or "scFv" is intended to refer to a molecule comprising an antibody heavy chain variable domain (or region; VH) and an antibody light chain variable domain (or region; VL) connected by a linker. Such scFv molecules can have the general structures: NH₂-VL-linker-VH-COOH or NH₂-VH-linker-VL-COOH. A suitable linker in the prior art is composed of a repetitive GGGGS amino acid sequence or a variant thereof, for example a variant with 1-4 repeat (Holliger et al. (1993),Proc. Natl. Acad. Sci. USA90: 6444-6448). Other linkers that may be used in the present invention are described by Alfthan et al., Protein Eng.8:725-731, Choi et al (2001), Eur.J.Immuno 1.31:94-106, Hu et al. (1996), Cancer Res.56:3055-3061, Kipriyanov et al. (1999), J.Mol.Biol.293:41-56 and Roovers et al (2001), Cancer Immunol.

The term "CDR" refers to one of the six hypervariable regions within the variable domains of an antibody that mainly contribute to antigen binding. One of the most commonly used definitions for the six CDRs was provided by Kabat E.A. et al, (1991) Sequences of proteins of immunological interest. NIH Publication 91-3242). As used herein, Kabat's definition of CDRs only applies for CDR1, CDR2 and CDR3 of the light chain variable domain (CDR L1, CDR L2, CDR L3, or L1, L2, L3), as well as for CDR2 and CDR3 of the heavy chain variable domain (CDR H2, CDR H3, or H2, H3).

The term "antibody framework", as used herein refers to the part of the variable domain, either VL or VH, which serves as a scaffold for the antigen binding loops (CDRs) of this variable domain. In essence it is the variable domain without the CDRs.

The term "epitope" or "antigenic determinant" refers to a site on an antigen (e.g., particular sites on thrombin molecule) to which an immunoglobulin or antibody specifically binds. An epitope typically includes at least 3, 4, 5, 6, 7, 8, 9, 10, 11, 12, 13, 14 or 15 consecutive or non-consecutive amino acids in a unique spatial conformation. See, e.g., Epitope Mapping Protocols in Methods in Molecular Biology, Vol. 66, G. E. Morris, Ed. (1996).

The terms "specific binding", "selective binding", "selectively binds", and "specifically binds" refer to binding of an antibody to an epitope on a predetermined antigen. Typically, the antibody binds with an affinity of approximately less than 10⁻⁷ M, such as approximately less than 10⁻⁸ M, 10⁻⁹ M or 10⁻¹⁰ M or even lower.

The term "KD" of "Kd" refers to the dissociation equilibrium constant of a particular antibody-antigen interaction. Typically, the antibodies of the invention bind to thrombin with a dissociation equilibrium constant (KD) of less than approximately 10⁻⁷ M, such as less than approximately 10⁻⁸ M, 10⁻⁹ M or 10⁻¹⁰ M or even lower, for example, as determined using surface plasmon resonance (SPR) technology in a BIACORE instrument.

The term "nucleic acid molecule" as used herein refers to DNA molecules and RNA molecules. A nucleic acid molecule may be single-stranded or double-stranded, but preferably is double-stranded DNA. A nucleic acid is "effectively linked" when it is placed into a functional relationship with another nucleic acid sequence. For instance, a promoter or enhancer is effectively linked to a coding sequence if it affects the transcription of the sequence.

The term "vector" refers to a nucleic acid molecule capable of transporting another nucleic acid to which it has been linked. In one embodiment, the type of vector is a "plasmid," which refers to a circular double stranded DNA loop into which additional DNA segments may be ligated. In another embodiment, the type of vector is a viral vector, wherein additional DNA segments may be ligated into the viral genome. In present invention, the vectors are capable of autonomous replication in a host cell into which they are introduced (e.g., bacterial vectors having a bacterial origin of replication and episomal mammalian vectors), or can be integrated into the genome of a host cell upon introduction into the host cell, and thereby are replicated along with the host genome (e.g., non-episomal mammalian vectors).

Methods for producing and purifying antibodies and antigen-binding fragments are well known in the art and can be found, for example, in Antibody Experimental Technology Guide of Cold Spring Harbor, Chapter 5-8 and 15. For example, mice can be immunized with human thrombin, or fragments thereof, and the resulting antibodies can then be renatured, purified and sequenced by using conventional methods well known in the art. Antigen-binding fragments can also be prepared by conventional methods. The antibody or the antigen-binding fragment of the present invention is genetically engineered to introduce one or more human framework regions (FRs) to a non-human derived CDR. Human FR germline sequences can be obtained from ImMunoGeneTics (IMGT) via their website http://imgt.cines.fr, or from The Immunoglobulin FactsBook, 2001ISBN012441351.

The term "host cell" refers to a cell into which an expression vector has been introduced. Host cells can include bacterial, microbial, plant or animal cells. Bacteria, which are susceptible to be transformed, include members of the enterobacteriaceae, such as strains of Escherichia coli or Salmonella; Bacillaceae, such as Bacillus subtilis; Pneumococcus; Streptococcus, and Haemophilus influenzae. Suitable microorganisms include Saccharomyces cerevisiae and Pichia pastoris. Suitable animal host cell lines include CHO (Chinese Hamster Ovary lines) and NS0 cells.

The engineered antibody or antigen-binding fragment of the present invention may be prepared and purified using conventional methods. For example, cDNA sequences encoding a heavy chain and a light chain may be cloned and recombined into a GS expression vector. The recombinant immunoglobulin expression vector may then stably transfect CHO cells. As a more recommended method well known in the art, mammalian expression systems will result in glycosylation, typically at the highly conserved N-terminus in the Fc region. Stable clones are obtained through expression of an antibody specifically binding to human thrombin. Positive clones may be expanded in a serum-free culture medium for antibody production in bioreactors. Culture medium, into which an antibody has been secreted, may be purified by conventional techniques. For example, the medium may be conveniently applied to a Protein A or G Sepharose FF column that has been equilibrated with a compatible buffer. The column is washed to remove nonspecific binding components. The bound antibody is eluted by PH gradient and antibody fragments are detected by SDS-PAGE, and then pooled. The antibody may be filtered and concentrated using common techniques. Soluble mixture and aggregate may be effectively removed by common techniques, including size exclusion or ion exchange. The obtained product may be immediately frozen, for example at -70°C, or may be lyophilized.

"Administration" and "treatment" as it applies to an animal, human, experimental subject, cell, tissue, organ, or biological fluid, refers to contacting an exogenous pharmaceutical, therapeutic, diagnostic agent, or composition with the animal, human, subject, cell, tissue, organ, or biological fluid. "Administration" and "treatment" can refer, e.g., to therapeutic, pharmacokinetic, diagnostic, research, and experimental methods. Treatment of a cell encompasses contacting a reagent with the cell, as well as contacting a reagent with a fluid, where the fluid is in contact with the cell. "Administration" and "treatment" also means in vitro and ex vivo treatments, e.g., of a cell, by a reagent, diagnostic, binding compound, or by another cell. "Treatment," as it applies to a human, veterinary, or a research subject, refers to therapeutic treatment, prophylactic or preventative measures, to research and diagnostic applications.

"Treat" means to administer a therapeutic agent, such as a composition comprising any of the binding compounds of the present invention, internally or externally to a patient having one or more disease symptoms for which the agent has known therapeutic activity. Typically, the agent is administered in an amount effective to alleviate one or more disease symptoms in the treated patient or population, whether by inducing the regression of or inhibiting the progression of such symptom(s) to any clinically measurable degree. The amount of a therapeutic agent that is effective to alleviate any particular disease symptom (also referred to "therapeutically effective amount") may vary according to factors such as the disease state, age, and weight of the patient, and the ability of the drug to elicit a desired response in the patient. Whether a disease symptom has been alleviated can be assessed by any clinical measurement typically used by physicians or other skilled healthcare providers to assess the severity or progression status of that symptom. While an embodiment of the present invention (e.g., a treatment method or article of manufacture) may not be effective in alleviating the disease symptom(s) of interest in every patient, it should alleviate the target disease symptom(s) of interest in a statistically significant number of patients as determined by any statistical test known in the art such as the Student's t-test, the chi-square test, the U-test according to Mann and Whitney, the Kruskal-Wallis test (H-test), Jonckheere-Terpstra-test and the Wilcoxon-test.

"Conservative modifications" or "conservative replacement or substitution" refers to substitutions of amino acids in a protein with other amino acids having similar characteristics (e.g. charge, side-chain size, hydrophobicity/hydrophilicity, backbone conformation and rigidity, etc.), such that the changes can frequently be made without altering the biological activity of the protein. Those of skill in this art recognize that, in general, single amino acid substitutions in non-essential regions of a polypeptide do not substantially alter biological activity (see, e.g., Watson et al. (1987) Molecular Biology of the Gene, The Benjamin/Cummings Pub. Co., p. 224 (4.sup.th Ed.)). In addition, substitutions of structurally or functionally similar amino acids are less likely to disrupt biological activity.

"Effective amount" encompasses an amount sufficient to ameliorate or prevent a symptom or sign of a medical condition. Effective amount also means an amount sufficient to allow or facilitate diagnosis. An effective amount for a particular patient or veterinary subject may vary depending on factors such as the condition being treated, the general health of the patient, the route and dose of administration and the severity of side effects. An effective amount can be the maximal dose or dosing protocol that avoids significant side effects or toxic effects.

"Exogenous" refers to substances that are produced outside an organism, cell, or human body, depending on the context. "Endogenous" refers to substances that are produced within a cell, organism, or human body, depending on the context.

"Homology" refers to sequence similarity between two polynucleotide sequences or between two polypeptides. When a position in both of the two sequences to be compared is occupied by the same base or amino acid monomer subunit, e.g., if a position in each of two DNA molecules is occupied by adenine, then the molecules are homologous at that position. The percent of homology between two sequences is a function of the number of matching or homologous positions shared by the two sequences divided by the number of positions compared multiplying by 100. For example, if 6 of 10 positions in two sequences are matched or homologous when the sequences are optimally aligned, then the two sequences are 60% homologous. If 95 of 100 positions in two sequences are matched or homologous when the sequences are optimally aligned, then the two sequences are 95% homologous. Generally, the comparison is made when two sequences are aligned to give maximum percent homology.

As used herein, the expressions "cell", "cell line" and "cell culture" are used interchangeably and all such designations include progeny. Thus, the words "transformants" and "transformed cells" include the primary subject cell and cultures derived therefrom without considering the number of transfers. It is also understood that all progeny may not be precisely identity in DNA content, due to deliberate or inadvertent mutations. Mutant progeny that have the same function or biological activity as screened for in the originally transformed cell are included. Where distinct designations are intended, it will be clear from the context.

As used herein, "polymerase chain reaction" or "PCR" refers to a procedure or technique in which minute amounts of a specific moiety of nucleic acid, RNA and/or DNA, are amplified as described in, e.g., U.S. Pat. No. 4,683,195. Generally, sequence information from the ends of, or beyond the region of interest needs to be available, such that oligonucleotide primers can be designed; these primers will be identity or similar in sequence to corresponding strands of the template to be amplified. The 5' terminal nucleotides of the two primers can be identity with the ends of the material to be amplified. PCR can be used to amplify specific RNA sequences, specific DNA sequences from total genomic DNA, and cDNA transcribed from total cellular RNA, bacteriophage or plasmid sequences, etc. See generally Mullis et al. (1987) Cold Spring Harbor Symp. Ouant. Biol. 51:263; Erlich, ed., (1989) PCR TECHNOLOGY (Stockton Press, N.Y.). As used herein, PCR is considered as one, but not the only, example of a nucleic acid polymerase reaction method for amplifying a nucleic acid test sample, comprising the use of a known nucleic acid as a primer and a nucleic acid polymerase to amplify or generate a specific moiety of the nucleic acid.

"Optional" or "optionally" means that the event or situation that follows may but does not necessarily occur, and the description includes the instances in which the event or circumstance does or does not occur. For example, "optionally comprises 1-3 antibody heavy chain variable regions" means the antibody heavy chain variable region with specific sequence can be, but not necessarily be present.

"Pharmaceutical composition" refers to one containing one or more compounds according to the present invention or a physiologically/pharmaceutically acceptable salt or prodrug thereof with other chemical components, as well as additional components such as physiologically/pharmaceutically acceptable carriers and excipients. The pharmaceutical composition aims at promoting the administration to an organism, facilitating the absorption of the active ingredient and thereby exerting a biological effect.

**EXAMPLE AND TEST**Hereinafter, the present invention is further described with reference to examples. However, the scope of the present invention is not limited thereto. In the examples of the present invention, where specific conditions are not described, the experiments are generally conducted under conventional conditions as described in Antibody Technology Laboratory Manual and Molecular Cloning Manual of Cold Spring Harbor, or under conditions proposed by the material or product manufacturers. Where the source of the reagents is not specifically given, the reagents are commercially available conventional reagents.

### Example 1: Preparation of thrombin antigen and detection protein

### 1. Related thrombin and prothrombin sequence

The sequence encoding human prothrombin (h-prothrombin), mouse prothrombin (m-prothrombin) and cynomolgus monkey prothrombin (cyno-prothrombin) were synthesized by CRO Shanghai Xu Guan Biological Science and Technology Development Co., Ltd. (all of the above prothrombin proteins have been designed by the inventors of the present invention). After one-step PCR with the His or Flag coding sequence at the 3' end, human prothrombin with His tag (h-prothrombin-his), human prothrombin with Flag tag (h-prothrombin-flag), mouse prothrombin with His tag (m-prothrombin-his), and cynomolgus monkey prothrombin with His tag (cyno-prothrombin-his) were cloned into the pTT5 vector (Biovector, Cat#: 102762), respectively, and then transiently expressed in 293 cells. Prothrombin obtained by recombinant expression was purified and activated in vitro to obtain thrombin, and further purified to obtain human thrombin with Flag tag (h-thrombin-flag) which was used for immunization, human thrombin with His tag (h-thrombin-His), mouse thrombin with His tag (m-thrombin-His) and cynomolgus monkey thrombin with His tag (cyno-thrombin-His) were used for screening *in vitro.*
SEQ ID NO: 1
   Human prothrombin with Flag tag (DYKDDDDK) 
MAHVRGLQLPGCLALAALCSLVHSQHVFLAPQQARSLLQRVRRANTFLEEVR
KGNLERECVEETCSYEEAFEALESSTATDVFWAKYTACETARTPRDKLAACLE
GNCAEGLGTNYRGHVNITRSGIECQLWRSRYPHKPEINSTTHPGADLQENFCR
NPDSSTTGPWCYTTDPTVRRQECSIPVCGQDQVTVAMTPRSEGSSVNLSPPLE
QCVPDRGQQYQGRLAVTTHGLPCLAWASAQAKALSKHQDFNSAVQLVENFC
RNPDGDEEGVWCYVAGKPGDFGYCDLNYCEEAVEEETGDGLDEDSDRAIEG
RTATSEYQTFFNPRTFGSGEADCGLRPLFEKKSLEDKTERELLESYIDGRIVEGS
DAEIGMSPWQVMLFRKSPQELLCGASLISDRWVLTAAHCLLYPPWDKNFTEN
 
DLLVRIGKHSRTRYERNIEKISMLEKIYIHPRYNWRENLDRDIALMKLKKPVAF
SDYIHPVCLPDRETAASLLQAGYKGRVTGWGNLKETWTANVGKGQPSVLQV
VNLPIVERPVCKDSTRIRITDNMFCAGYKPDEGKRGDACEGDSGGPFVMKSPF
NNRWYQMGIVSWGEGCDRDGKYGFYTHVFRLKKWIQKVIDQFGEDYKDDD
DK

SEQ ID NO: 2
 Human prothrombin with His tag (HHHHHH) 
MAHVRGLQLPGCLALAALCSLVHSQHVFLAPQQARSLLQRVRRANTFLEEVR
KGNLERECVEETCSYEEAFEALESSTATDVFWAKYTACETARTPRDKLAACLE
GNCAEGLGTNYRGHVNITRSGIECQLWRSRYPHKPEINSTTHPGADLQENFCR
NPDSSTTGPWCYTTDPTVRRQECSIPVCGQDQVTVAMTPRSEGSSVNLSPPLE
QCVPDRGQQYQGRLAVTTHGLPCLAWASAQAKALSKHQDFNSAVQLVENFC
RNPDGDEEGVWCYVAGKPGDFGYCDLNYCEEAVEEETGDGLDEDSDRAIEG
RTATSEYQTFFNPRTFGSGEADCGLRPLFEKKSLEDKTERELLESYIDGRIVEGS
DAEIGMSPWQVMLFRKSPQELLCGASLISDRWVLTAAHCLLYPPWDKNFTEN
DLLVRIGKHSRTRYERNIEKISMLEKIYIHPRYNWRENLDRDIALMKLKKPVAF
SDYIHPVCLPDRETAASLLQAGYKGRVTGWGNLKETWTANVGKGQPSVLQV
VNLPIVERPVCKDSTRIRITDNMFCAGYKPDEGKRGDACEGDSGGPFVMKSPF
NNRWYQMGIVSWGEGCDRDGKYGFYTHVFRLKKWIQKVIDQFGEHHHHHH

SEQ ID NO: 3
 cynomolgus monkey prothrombin with His tag 
MAHVRGLQLPGCLALAALCSLVHSQHVFLAPQQALSLLQRVRRASSGFLEEV
FKGNLERECVEETCSYEEAFEALESSTATDAFWAKYTACETARTSRDTLAACL
EGNCAEDLGTNYRGHVNITRSGIECQLWRSRYPHKPEINSTTHPGADLQENFC
RNPDSSTTGPWCYTTDPTVRREECSIPVCGQDQVTVVMTPRSSVNLSLPSEEC
VPDRGRQYQGHLAVTTHGLPCLAWASAQAKALSKHQDFDSAVQLVENFCRN
PDGDEEGVWCYVAGKPGDFEYCDLNYCEEAVDEETGDGLGEDPDRAIEGRTA
TSEYQTFFDPRTFGLGEADCGLRPLFEKKSLEDKTEGELLESYIDGRIVEGWD
AEIGMSPWQVMLFRKSPQELLCGASLISDRWVLTAAHCLLYPPWDKNFTEND
LLVRIGKHSRTRYERNIEKISMLEKIYIHPRYNWRENLDRDIALMKLKKPITFSD
YIHPVCLPDRETAASLFQAGYKGRVTGWGNLKETWTTNVGKVQPSVLQVVN
LPIVERSVCKDSTRIRITDNMFCAGYKPGEGKRGDACEGDSGGPFVMKNPLN
KRWYQMGIVSWGEGCDRDGKYGFYTHVFRLKKWIQKVIDQFGDHHHHHH

SEQ ID NO: 4
 mouse prothrombin with His tag

MSHVRGLGLPGCLALAALVSLVHSQHVFLAPQQALSLLQRVRRANSGFLEEL
RKGNLERECVEEQCSYEEAFEALESPQDTDVFWAKYTVCDSVRKPRETFMDC
LEGRCAMDLGVNYLGTVNVTHTGIQCQLWRSRYPHKPEINSTTHPGADLKEN
FCRNPDSSTTGPWCYTTDPTVRREECSVPVCGQEGRTTVVMTPRSGGSKDNL
SPPLGQCLTERGRLYQGNLAVTTLGSPCLPWNSLPAKTLSKYQDFDPEVKLVE
NFCRNPDWDEEGAWCYVAGQPGDFEYCNLNYCEEAVGEENYDVDESIAGRT
TDAEFHTFFNEKTFGLGEADCGLRPLFEKKSLKDTTEKELLDSYIDGRIVEGW
DAEKGIAPWQVMLFRKSPQELLCGASLISDRWVLTAAHCILYPPWDKNFTEN
DLLVRIGKHSRTRYERNVEKISMLEKIYVHPRYNWRENLDRDIALLKLKKPVP
FSDYIHPVCLPDKQTVTSLLRAGYKGRVTGWGNLRETWTTNINEIQPSVLQV
VNLPIVERPVCKASTRIRITDNMFCAGFKVNDTKRGDACEGDSGGPFVMKSPF
NNRWYQMGIVSWGEGCDRKGKYGFYTHVFRLKRWIQKVIDQFGHHHHHH

Note: The double-underlined part of the sequence is the thrombin sequence. The single underlined part of the sequence is the corresponding tag sequence.

### 2. Purification and activation in vitro of prothrombin-associated recombinant proteins, purification of thrombin related recombinant protein, hybridoma antibody and recombinant antibody

### 1). Purification steps of human prothrombin with His tag, mouse prothrombin with His tag and cynomolgus monkey prothronbin with His tag

Cell supernatant sample was centrifuged at high speed to remove impurities. After that, the nickel column was equilibrated with PBS buffer (pH 7.4), washed with 2-5 column volumes, and the supernatant sample was loaded onto the Ni Sepharose excel column at a flow rate. Washing the column with PBS until the reading at A280 was reduced to baseline. Then, the column was washed with PBS+10mM imidazole, and the non-specifically binding impure protein was washed off and then the effluent was collected. Finally, the target protein was eluted with PBS buffer containing 300mM imidazole and the elution peak was collected. The collected eluent was concentrated and the sample buffer was exchanged with PBS solution using a desalting column for subsequent activation in vitro and further purification.

### 2). Purification steps of recombinant human prothrombin protein with Flag tag:

The sample was centrifuged at high speed to remove impurities and concentrated to an appropriate volume. After that, the flag affinity column was equilibrated by 0.5×PBS (pH7.4) and washed with 2-5 column volumes. The supernatant samples were loaded onto the column after removing the impurity. Washing the column with 0.5×PBS until the reading at A280 was reduced to baseline. Then, the column was washed with PBS, and the impure protein was washed off and then collected. The target protein was eluted with TBS buffer containing 100µg/ml of 3×Flag polypeptide and collected for further activation and purification in vitro.

### 3). In vitro activation of prothrombin related recombinant protein and further purification of the activated thrombin related protein.

The prothrombin was obtained by the preliminary affinity chromatography (nickel column or Flag affinity column), and mixed evenly with the Ecarin enzyme at a mass ratio of 100:1 and was incubated overnight at room temperature. The activated sample was centrifuged at high speed to remove the precipitate and further purified using an ion exchange column and size exclusion chromatography SEC.

### 3.1) Ion exchange

Human thrombin with His tag, mouse thrombin with His tag and cynomolgus monkey with His tag were purified by the cationic exchange column SP HP column. Human thrombin with Flag tag was further purified using an anion exchange column Q HP column. The cationic exchange buffer A was 10 mM PB, pH 6.8 buffer, buffer B was 10 mM PB, pH 6.8, 1 M NaCl buffer. For anion exchange, buffer A was 10 mM Tris-HCl, pH 8.5 buffer and buffer B was 10 mM Tris-HCl, pH 8.5, 1 M NaCl buffer.

The ion exchange column was pre-equilibrated with 5 column volumes buffer A. The activated thrombin samples exchanged with buffer A were loaded at a flow rate. At the end of loading, equilibrating the column with buffer A until the reading at A280 was reduced to baseline. The elution peaks were collected by gradient elution from 0% to 80% in 20 column volumes using buffer B. The components of the target protein were identified by SDS-PAGE and further verified by mass spectrometry and peptide mapping.

### 3.2) Size exclusion chromatography

The SEC column (superdex75) was pre-equilibrated with PBS (pH 7.4). The sample was loaded and eluted with PBS as the mobile phase, eluted peaks were collected. The components of the target protein were identified by SDS-PAGE and further verified by mass spectrometry and peptide mapping.

### 3.3) Purification of hybridoma and recombinant antibody

Cell supernatant sample was centrifuged at high speed to remove impurities. Hybridoma expression supernatant was purified by Protein G column, and recombinant antibody expression supernatant was purified by Protein A column. Washing the column with PBS (7.4) until the reading at A280 was reduced to baseline. The target protein was eluted with 100 mM acetic acid pH 3.0 and neutralized with 1 M Tris-HCl, pH 8.0. The eluted sample was properly concentrated and further purified using gel chromatography Superdex200 (GE), which was equilibrated with PBS, the aggregate peak was excluded and the monomer peak was collected and aliquoted for use.

### Example 2: Preparation of Anti-human thrombin monoclonal antibody

### 1. Immunization

The anti-human thrombin monoclonal antibody was produced by immunizing mice. Experimental SJL white mice, female, 6-week old (Beijing Weitong Lihua Experimental Animal Technology Co., Ltd., animal production license number: SCXK (Beijing) 2012-0001). Feeding environment: SPF level. After the mice were purchased, the animals were kept in the laboratory for 1 week, with 12/12-hour light/dark cycle, at temperature of 20-25°C, and with a humidity of 40-60%. The mice that had been adapted to the environment were immunized according to the following scheme. Immune antigen was human thrombin with Flag tag (as shown in double underline of SEQ ID NO: 1).

Immunization Scheme: Mice were cross-immunized with TiterMax® Gold Adjuvant (Sigma Cat No.: T2684) and Thermo Imject® Alum (Thremo Cat No.: 77161). The ratio of antigen to adjuvant (TiterMax® Gold Adjuvant) was 1:1, and the ratio of antigen to adjuvant (Thermo Imject® Alum) was 3:1, with a dose of 50µg/mouse (first immunization) and 25µg/mouse (booster immunization). After the antigen was emulsified, the mice were inoculated on day 0, 14, 28, 42 and 56.

On day 0, the mice were intraperitoneally (i.p.) injected with emulsified antigen, 50µg/mouse. On day 14, the mice were subcutaneously (s.c.) injected with 25µg/mouse at multiple sites (usually 6-8 sites on the back). On days 28 and 42, either back or intraperitoneal injection of antigen was selected according to the lumps on the back and the swelling conditions in abdomen. A booster immunization was performed by i.p. injection of antigen solution formulated with saline, 50µg/mouse, 3 days prior to splenocyte fusion.

Blood samples were collected on days 22, 36, 50, 64, and antibody titers in mouse serum were determined by ELISA. After 4 immunizations, mice with higher antibody titer and the titer tending to platform in their serum were selected for splenocyte fusion.

### 2. Splenocyte Fusion

Hybridoma cells were obtained by fusing splenic lymphocytes with myeloma Sp2/0 cells (ATCC® CRL-8287™) by using an optimized PEG-mediated fusion procedure. The hybridoma cells obtained were resuspended in a MC semi-solid complete medium (RPMI-1640 medium containing 20% FBS, 1×HAT, 1×OPI and 2% Methylcellulose) at a density of 0.5-1×10⁶/ml, and incubated in 35mm cell culture plates, incubation at 37°C, 5%CO₂, for 7-9 days. On days 7-9 after fusion, single cell clones were picked into 96-well cell culture plate containing 200µl/well of HT complete medium (RPMI-1640 medium containing 20% FBS, 1×HAT and 1×OPI), cultured at 5% CO₂ at 37°C for three days and then detected by ELISA assay.

### 3. Screening for Hybridoma Cells

Hybridoma culture supernatant was detected by thrombin binding ELISA (see Test1) according to the growth density of hybridoma cells. And prothrombin binding ELISA test was performed with the positive cells during the above binding ELISA (see Test3). Positive cells were expanded and frozenly stored timely, and the cells were subcloned twice to three times until single cell clone was obtained.

After each subcloning procedure, the cells were subjected to thrombin binding ELISA and prothrombin binding ELISA. The hybridoma clone mAb-1601 was obtained by the above screening experiments, and the antibody was further prepared by serum-free cell culture method, and then the antibody was purified by purification example for use in the test examples.

### 4. Sequencing of the positive hybridoma clone

The process of cloning sequence from the positive hybridoma was as follows: Collecting the hybridoma cells at logarithmic growth phase, and extracting RNA with Trizol (Invitrogen Cat, No. 15596-018) according to the kit instructions, and then performing reverse transcription with the PrimeScript™ Reverse Transcriptase kit (Takara, Cat No. 2680A). The cDNAs obtained by reverse transcription were amplified by PCR using the mouse Ig-Primer Set (Novagen, TB326 Rev.B 0503) and sequencing was performed in a sequencing company.

The amino acid sequences corresponding to DNA sequences of positive clone mAb-1601 are shown in SEQ ID NO: 5 and SEQ ID NO: 6, respectively.
Heavy chain variable region obtained from hybridoma: 
EVKLVESEGGLVQPGSSLKLSCTASGFTFSDYYMAWVRQVPEKGLEWVANINSD
GSSTYYLDSLKSRFIISRDNAKNILYLQMSSLKSEDTATYYCARDHYHGNSYVFD
YWGQGTTLTVSS

SEQ ID NO:5
Light chain variable region obtained from hybridoma:

DIQMTQSSSSFSVSRGNRVTITCKASEDIYNRLAWYQQKPGNAPRLIISGATSLET
GVPSRFSGSGSGKDYTLSITSLQTEDVGTYYCQQYWSTPWTFGGGTKLEIK

SEQ ID NO:6

NOTE: The order is FR1-CDR1-FR2-CDR2-FR3-CDR3-FR4, italic sequence represents FR sequence, and underlined sequence represents CDR sequence.

**Table 1 CDR region sequences of heavy chain and light chain**

| | Heavy Chain | | Light Chain | |
|---|---|---|---|---|
| mAb-1601 | HCDR1 | DYYMA SEQ ID NO:7 | LCDR1 | KASEDIYNRLA SEQ ID NO:10 |
| | HCDR2 | NINSDGSSTYYLDSL KS SEQ ID NO:8 | LCDR2 | GATSLET SEQ ID NO:11 |
| | HCDR3 | DHYHGNSYVFDY SEQ ID NO:9 | LCDR3 | QQYWSTPWT SEQ ID NO:12 |

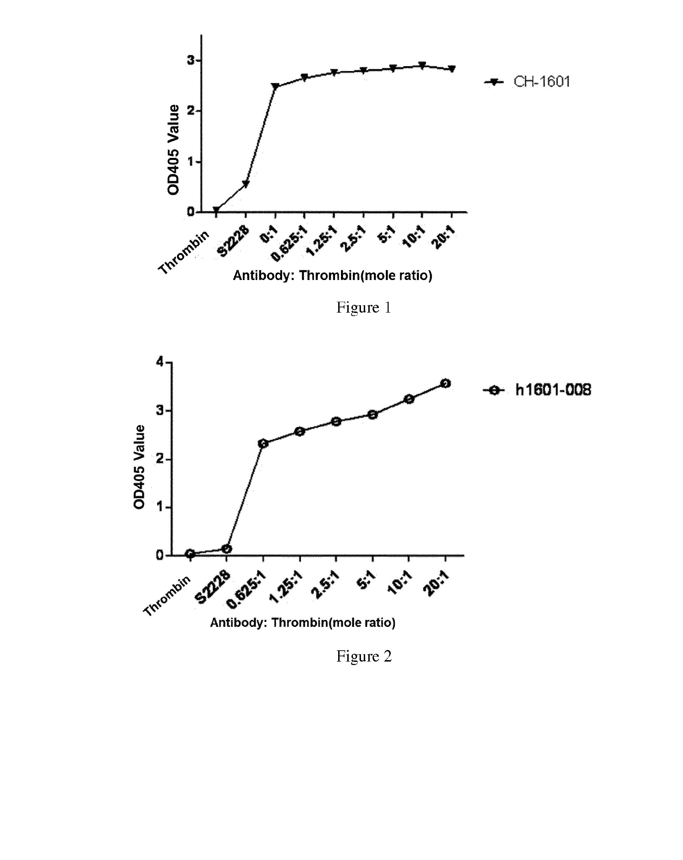

The positive clone mAb-1601 was cloned and expressed in Example 4 to obtain the chimeric antibody CH-1601. The antibody was identified for binding activity against human thrombin and prothrombin (Test 1 and Test 2). At the same time, the chimeric antibody CH-1601 was detected for its cross-binding activity of with thrombin of the genus monkey (see Test 3), affinity with human thrombin (see Test 6), and influence on human thrombin activity (see test examples 5). The detection results are shown in the following table 2 and figure 1:

**Table 2. In vitro activity of chimeric antibody binding to different species of thrombin and human prothrombin**

| | Chimeric Antibody CH-1601 | |
|---|---|---|
| Substrate | Binding ELISA EC50(nM) | BIAcore KD(nM) |
| Human thrombin | 0.71 | 2.27 |
| Monkey thrombin | 9.98 | 3.13 |
| murine thrombin | no binding | no binding |
| Human prothrombin | 42.6 | 78.7 |

Table 2 shows that CH-1601 has strong binding capacity with thrombin of different species, including human and monkey thrombin, but not binding with murine thrombin. Figure 1 shows that the binding of CH-1601 to thrombin does not affect its enzymatic activity on substrate S2228.

### Example 3. Humanization of murine anti-human thrombin monoclonal antibody

### 1. Selection of a framework for humanizing hybridoma clone mAb-1601

Through comparison in the IMGT human antibody heavy and light chain variable region gene database and MOE software, the heavy and light chain variable region germline genes with high homology to mAb-1601 was selected as templates, the CDRs derived from the two murine antibodies were grafted into the corresponding human source template to form a variable region sequence with the order in FR1-CDR1-FR2-CDR2-FR3-CDR3-FR4. Wherein, amino acid residues were identified and annotated by the Kabat Numbering System.

The light chain template for humanizing murine antibody mAb-1601 is IGKV1-39*01 and hjk4.1, the heavy chain template for humanization is IGHV3-23*04 and hjh6.1, the sequences of humanized variable region are as follows:
> h1601-001 (CDR graft), humanized heavy chain variable region 
EVQLVESGGGLVQPGGSLRLSCAASGFTFSDYYMAWVRQAPGKGLEWVSNINSD
GSSTYYLDSLKSRFTISRDNSKNTLYLQMNSLRAEDTAVYYCAKDHYHGNSYVFD
YWGQGTTVTVSS

SEQ ID NO:13
> h1601-001 (CDR graft) humanized light chain variable region

DIQMTQSPSSLSASVGDRVTITCKASEDIYNRLAWYQQKPGKAPKLLIYGATSLET

GVPSRFSGSGSGTDFTLTISSLQPEDFATYYCQQYWSTPWTFGGGTKVEIK

SEQ ID NO: 16

Note: the order is FR1-CDR1-FR2-CDR2-FR3-CDR3-FR4, the italic sequence is FR sequence, and the underlined is CDR sequence.

### 2. Back-mutation design of hybridoma clone CH-1601, see Table 3 below:

**Table 3. Back-mutation design of hybridoma clone**

| **h1601_VL** | | **h1601_VH** | |
|---|---|---|---|
| h1601_VL.1 | Graft | h1601_VH.1 | Graft |
| h1601_VL.1A | Y49S, T69K | h1601_VH.1A | S49A |
| h1601_VL.1B | Y49S, T69K, K45R, L47I | h1601_VH.1B | R87K, K98R |
| h1601_VL.1C | Y49S, T69K, K45R, L47I, F71Y | h1601_VH.1C | Q3K, |
| | | h1601_VH.1D | R87K, K98R, Q3K, S49A |

NOTE: For example, Q3K denotes a back mutation from Q toK at position 3 according to Kabat numbering system. Grafted indicates that the murine antibody CDR was implanted into human germline FR sequences.

The specific sequences after the mutation are shown as follows:
h1601_VH.1B: 
EVQLVESGGGLVQPGGSLRLSCAASGFTFSDYYMAWVRQAPGKGLEWVSNI
NSDGSSTYYLDSLKSRFTISRDNSKNTLYLQMNSLKAEDTAVYYCARDHYHG
NSYVFDYWGQGTTVTVSS

SEQ ID NO:14
h1601_VH.1D: 
EVKLVESGGGLVQPGGSLRLSCAASGFTFSDYYMAWVRQAPGKGLEWVANI
NSDGSSTYYLDSLKSRFTISRDNSKNTLYLQMNSLKAEDTAVYYCARDHYHG
NSYVFDYWGQGTTVTVSS
 SEQ ID NO:15
h1601_VL.1A: 
DIQMTQSPSSLSASVGDRVTITCKASEDIYNRLAWYQQKPGKAPKLLISGATSL
ETGVPSRFSGSGSGKDFTLTISSLQPEDFATYYCQQYWSTPWTFGGGTKVEIK

SEQ ID NO:17
h1601_VL.1C:

DIQMTQSPSSLSASVGDRVTITCKASEDIYNRLAWYQQKPGKAPRLIISGATSL
ETGVPSRFSGSGSGKDYTLTISSLQPEDFATYYCQQYWSTPWTFGGGTKVEIK
SEQ ID NO:18

The variable region of light and heavy chain after back-mutation is recombined, and the light and heavy chain variable region combinations included in the obtained antibodies are as follows:

**Table 4. Light chain and heavy chain variable region sequences of humanized antibody**

| Antibody Number | Heavy chain variable region | Light chain variable region |
|---|---|---|
| **h1601-001** | SEQ ID NO:13 | SEQ ID NO:16 |
| **h1601-002** | SEQ ID NO:14 | SEQ ID NO:16 |
| **h1601-003** | SEQ ID NO:15 | SEQ ID NO:16 |
| **h1601-004** | SEQ ID NO:13 | SEQ ID NO:17 |
| **h1601-005** | SEQ ID NO:14 | SEQ ID NO:17 |
| **h1601-006** | SEQ ID NO:15 | SEQ ID NO:17 |
| **h1601-007** | SEQ ID NO:13 | SEQ ID NO:18 |
| **h1601-008** | SEQ ID NO:14 | SEQ ID NO:18 |
| **h1601-009** | SEQ ID NO:15 | SEQ ID NO:18 |

The above variable regions were cloned and purified according to the preparation method of Example 4 below, and the binding activity of the antibody with human thrombin protein was detected by the ELISA of Test 1. According to the test results, antibodies were selected for further in vivo or in vitro activity detection.

### Example 4. Preparation of chimeric antibody and humanized antibody

The antibody was constructed with constant region derived from human heavy chain IgG4/light chain kappa in combination with each variable region, and a S228P mutation was made in Fc to increase the stability of the IgG4 antibody. The other mutations known in the art can also be used to increase its performance.
heavy chain constant region: 
ASTKGPSVFPLAPCSRSTSESTAALGCLVKDYFPEPVTVSWNSGALTSGVHTFP
AVLQSSGLYSLSSVVTVPSSSLGTKTYTCNVDHKPSNTKVDKRVESKYGPPCP
PCPAPEFLGGPSVFLFPPKPKDTLMISRTPEVTCVVVDVSQEDPEVQFNWYVD
GVEVHNAKTKPREEQFNSTYRVVSVLTVLHQDWLNGKEYKCKVSNKGLPSSI
EKTISKAKGQPREPQVYTLPPSQEEMTKNQVSLTCLVKGFYPSDIAVEWESNG
QPENNYKTTPPVLDSDGSFFLYSRLTVDKSRWQEGNVFSCSVMHEALHNHYT
QKSLSLSLGK

SEQ ID NO:19
light chain constant region:




SEQ ID NO:20

### 1. Molecular cloning of the chimeric antibody

The variable region coding sequences were obtained by sequencing the candidate antibody molecules obtained from hybridoma screening. The primers were designed according to the obtained sequence, the sequencing gene was used as template, and various antibody VH/VK gene fragments were constructed by PCR, and then reconstituted with the expression vector pHr (with a signal peptide and hIgG4 constant region (CH1-FC/CL) fragment) to construct a full-length expression plasmid VH-CH1-FC-pHr/VL-CL-pHr for recombinant antibody.

### 2. Molecular cloning of humanized antibody

The coding sequence of human genetic code preference was generated after the genetic code optimization of the designed humanized antibody sequence. Primers were designed and the VH/VK gene fragments of the antibodies were constructed by PCR, and reconstituted with the expression vector pHr (with a signal peptide and constant region (CH1-FC/CL) fragment) to construct a full-length expression plasmid VH-CH1-FC-pHr/VL-CL-pHr for humanized antibody.

### 3. Expression and purification of chimeric and humanized antibody

The plasmids for expression of antibody light chain and heavy chain were co-transfected into HEK293E cell at a ratio of 1: 1.5. The expression supernatant was collected after 6 days and impurities were removed by high-speed centrifugation and then purified with Protein A column. The column was washed with PBS until the reading at A280 was reduced to baseline. The target protein was eluted with 100mM acetic acid, pH 3.0, and neutralized with 1M Tris-HCl, pH 8.0. The eluent was properly concentrated and further purified by gel chromatography Superdex200 (GE) which was equilibrated with PBS. The aggregate peak was excluded and the monomer peak was collected. Then the correct sample was aliquoted and for use.

### Example 5. Determination of antigen epitope

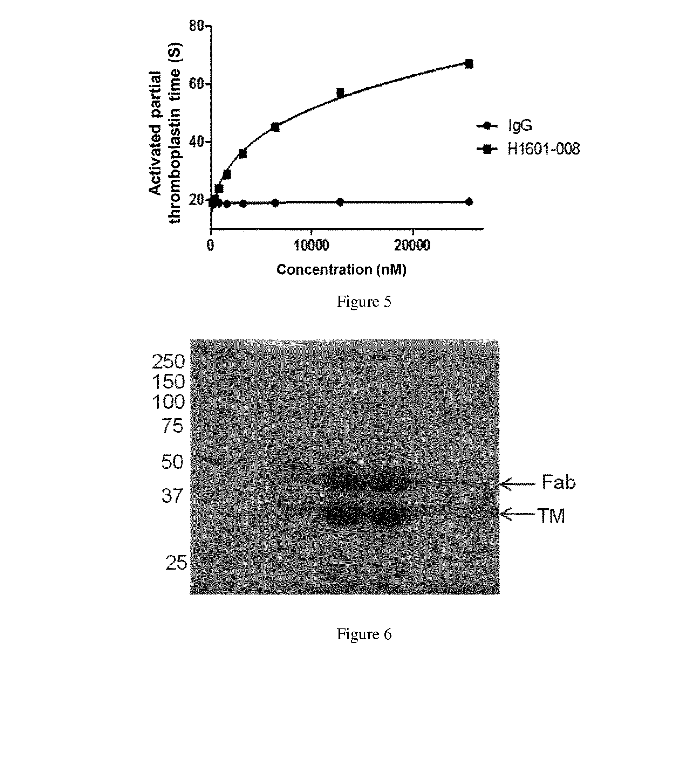

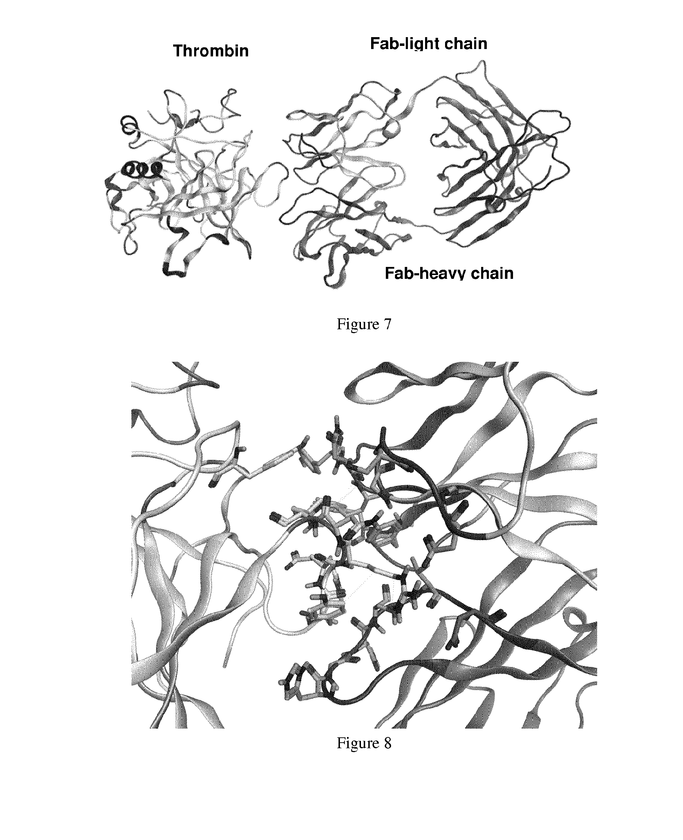

Purified antibody H1601-008 was cleaved with Papain (Figure 6). Fab fragments were separated and combined with human PPACK-thrombin. In which, PPACK is a thrombin inhibitor and purchased from ApexBio. Thrombin is the double-underlined sequence in SEQ ID NO: 1 of the patent. Human PPACK-thrombin-FAB complex was crystallized and used for structural analysis. The structural statistics obtained are as follows: the resolution is Rfactor = 23.4%, Rfree = 27.2%, and there are two complexes in the asymmetric unit, Ramachandran:favoured = 93.5%, and the abnormal value = 0%. The crystal structure shows that LCDR1, LCDR2, LCDR3 and HCDR3 of Fab are tightly bound to thrombin (Figure. 7).
Specifically, the R62, Y71, E72, R73, Y114 residues of thrombin have hydrogen-bond interactions with the four rings of the Fab (Figure. 8). The numbering rule for the thrombin residue numbers in this example is according to sequentially numbering from the N-terminus to the C-terminus of the thrombin fragment sequence of the present invention.

Analysis of antibody-thrombin-linked PISA indicated that the total hidden surface area in the complex was 803Å². The contact residues in the heavy chain are (Kabat numbering system): 100, 102, 103, 104, 105, 107, 109, which are all in CDR. CDRL1-KASEDIYNRLA, CDRL2-GATSLET, CDRL3-QQYWSTPWT, CDRH3-DHYHGNSYVFDY(Contact residues are underlined). CDRL was found to be more important, providing 57.3% of the hidden surface area of the antibody. The contact residues in light chains are 30, 31, 32, 46, 49, 50, 53, 55, 91, 92, 96.

The contact residues in thrombin are 9, 24, 60, 62, 64, 66, 69, 70, 71, 72, 73, 74, 75, 78, 113, 114. The most important independent contributions to the hidden surface area are peptide R70-Y71-E72-R73-N74-I75 and Y114.

### Example 6. Improve h1601-008 selection activity

We decided to carry out the affinity maturation against h1601-008, and to reduce or maintain its binding activity to pro-thrombin as much as possible while increase the affinity of thrombin, so as to increase the selectivity of h1601-008 between the two molecules.

M13 phage display technology was used to increase the selectivity. Codon-based primers (during the synthesis of primers, single codon consists of wild-type codon and NNK) were adopted to introduce mutations in each CDR, and a separate phage display library was constructed for each CDR. Based on the length of the CDRs, the ratio of NNK and the library size required for the library were adjusted. The specific plan is shown in Table 5.

**Table 5. Library size and NNK incorporation ratio**

| Lib | CDR length | NNK ratio | Lib size |
|---|---|---|---|
| H1 | 5 | 50% | >2E7 |
| H2 | 17 | 20% | >1E8 |
| H3 | 14 | 20% | >1E8 |
| L1 | 11 | 30% | >1E8 |
| L2 | 7 | 50% | >1E8 |
| L3 | 7 | 50% | >1E8 |

The constructed 6 libraries were packaged into phages for panning: pre-bound to high concentration of pro-thrombin in liquid phase, and then associated with biotinylated thrombin, captured by streptavidin, elutriated, eluted, then re-infected with E.coli for the next round of panning. The concentration of biotinylated thrombin was reduced by 2-5 fold in each round of panning. After 3 rounds of panning, a single clone was picked from each library for sequencing verification. It was found that only HCDR3 has obvious amino acid enrichment. According to the enrichment degree of amino acid residues in CDR regions, some clones such as aTM-1, aTM-2, aTM-4, aTM-7 were selected to construct full-length Ig for expression in mammalian cells.

The selected clone differed from h1601-008 only on HCDR3. The related HCDR3 formula and its corresponding heavy chain variable regions are described below.

The formula of HCDR3 sequence obtained by screening is as follows:
DHYX₁G X₂SYVFD X₃ SEQ ID NO:21;

Further, the related general formula of heavy chain variable region sequence was obtained as folllows: 
EVQLVESGGGLVQPGGSLRLSCAASGFTFSDYYMAWVRQAPGKGLEWVSNI
NSDGSSTYYLDSLKSRFTISRDNSKNTLYLQMNSLKAEDTAVYYCARDHYX1G
X2SYVFDX3WGQGTTVTVSS SEQ ID NO:22;
 wherein, X₁ is selected from the group consisting of H, I, L, M; X₂ is selected from N, A; X₃ is selected from Y, S, L,T.

Specific related sequences obtained include but are not limited to those described in Table 6.

**Table 6. Variable region sequence of heavy chain determined by affinity screening**

| variable region of heavy chain | X₁ | X₂ | X₃ | VH sequence number | HCDR3 sequence included |
|---|---|---|---|---|---|
| h1601-008 | H | N | Y | SEQ ID NO:14 | DHYHGNSYVFDY(SEQ ID NO:9) |
| aTM-1 | I | A | Y | SEQ ID NO:27 | DHYIGASYVFDY(SEQ ID NO:23) |
| aTM-2 | L | N | S | SEQ ID NO:28 | DHYLGNSYVFDS(SEQ ID NO:24) |
| aTM-4 | L | N | L | SEQ ID NO:29 | DHYLGNSYVFDL(SEQ ID NO:25) |
| aTM-7 | M | N | T | SEQ ID NO:30 | DHYMGNSYVFDT(SEQ ID NO:26) |

After the cloned protein was purified, the affinity to Thrombin and pro-thrombin was measured using biacore.

The result showed that the selectivity of aTM-1 was increased by 1.8 times (18.2/9.9), and the affinity to Thrombin was increased significantly, while the affinity to pro-thrombin was also increased. The results are shown in table 10.

The performance and benefits of the antibodies of the present invention are verified by biochemical test methods as below.

### Test 1. ELISA assay for binding of the thrombin antibodies to human thrombin protein

The binding ability of anti-thrombin antibody was detected by ELISA assay with the antibodies and the human thrombin with His tag. Thrombin protein with His tag labeled with the biotin-labeling kit (Dongren Chemicals, Cat No. LK03) was immobilized into 96-well microtiter plate by binding to streptavidin coated on the microtiter plate, the strength of the signal after the addition of the antibody was used to determine the binding activity of the antibody to thrombin. The specific experimental method is as follows.

Streptavidin (Sigma, Cat No. S4762-5MG) was diluted to a concentration of 5µg/ml with PBS buffer, pH 7.4 (Sigma, Cat No. P4417-100TAB), and added to a 96-well plate at a volume of 50µl/well and then, the plate was incubated in the incubator at 37°C for 2 hours. After discarding the liquid, the plates were blocked with 200µl/well of blocking solution containing 5% skim milk (Guangming skim milk) in PBS, and incubated in the incubator at 37°C for 2.5 hours or overnight at 4°C (16-18 hours). After blocking, the blocking solution was discarded and the plate was washed 5 times with PBST buffer (PBS containing 0.05% tweeen-20, pH7.4). Human thrombin protein with His tag labeled with the biotin-labeling (SEQ ID NO: 2, as shown by the double underline) was diluted with sample dilution (PBS containing 1%BSA, PH7.4) to 50µg/ml and was added to each well, 50µl/well. Then the plate was incubated in the incubator at 37°C for 2h or overnight. After incubation, the blocking solution in the plate was discarded, and the plate was washed with PBST for 6 times, and then was added with 50µl/well of various concentrations of the test antibodies diluted with sample dilution and the plate was incubated at 37°C for 2h. The plates was washed 5 times with PBST after incubation, and was added with 100µl/well of goat anti-human secondary antibody (Jackson Immuno Research, Cat No. 109-035-003) labeled with HRP, diluted in sample dilution, and the plate was incubated at 37°C for 1h. After washing the plates 5 times with PBST, 50µl/well of TMB chromogenic substrate (KPL, Cat No. 52-00-03) was added to each well, and incubated at room temperature for 10-15 min, the reaction was stopped by the addition of 50µl/well 1M H₂SO₄ to each well. The OD value at a wavelength of 450nm was read on NOVOStar microplate reader, and then EC50 values of the binding of thrombin antibody to human thrombin were calculated. The results are shown in Table 7

**Table 7. Binding activity of chimeric antibody and humanized antibody**

| Humanized antibody | Binding ELISA EC50(nM) |
|---|---|
| h1601-005 | 5.80 |
| h1601-006 | 4.45 |
| h1601-008 | 1.54 |
| h1601-009 | 1.49 |

The data showed that all the humanized antibodies obtained by the screening method in the present invention showed excellent binding activities to human thrombin protein.

### Test 2. ELISA assay for binding of the thrombin antibodies to human pro-thrombin protein

Prothrombin is a precursor of thrombin, and thrombin is produced from the enzymolysis and activation of prothrombin. The binding ability of the anti-thrombin antibodies to h-prothrombin was detected by ELISA. The specific experimental method is as follows.

Human prothrombin with His tag (SEQ ID NO:2) was diluted to a concentration of 10µg/ml with PBS buffer, pH 7.4 (Sigma, Cat No. P4417-100TAB), and added to a 96-well plate at a volume of 100µl/well and then, the plate was incubated at 4°C for overnight. After discarding the liquid, the plates were blocked with 200µl/well of blocking solution containing 5% skim milk (Guangming skim milk) in PBS, and incubated in the incubator at 37°C for 2.5 hours. After blocking, the blocking solution was discarded and the plate was washed 5 times with PBST buffer (PBS containing 0.05% tweeen-20, pH7.4), and then was added with 50µl/well of the test antibodies gradient diluted with sample dilution (PBS containing 1% BSA, pH7.4) and the plate was incubated at 37°C for 1h. The plates was washed 5 times with PBST after incubation, and was added with 100µl/well of goat anti-human secondary antibody (Jackson Immuno Research,Cat No. 109-035-003) labeled with HRP, diluted in sample dilution, and the plate was incubated at 37°C for 1h. After washing the plates 5 times with PBST, 50µl/well of TMB chromogenic substrate (KPL, Cat No. 52-00-03) was added to each well, and incubated at room temperature for 10-15 min, the reaction was stopped by the addition of 50µl/well 1M H₂SO₄ to each well. The OD value at a wavelength of 450nm was read on NOVOStar microplate reader, and then EC50 values of the binding of the thrombin antibodies to human prothrombin were calculated.

### Test 3. Cross-binding assay for binding of the thrombin antibodies to different species thrombin

To test the in vitro binding ability of the screened thrombin antibody against monkey-derived thrombin, cynomolgus monkey thrombin was used for in vitro binding assays.

His-tag antibody (GenScript, Cat No. A00174) was diluted to a concentration of 0.5µg/ml with PBS buffer, pH 7.4 (Sigma, Cat No. P4417-100TAB), and added to a 96-well plate at a volume of 100µl/well and then, the plate was incubated in the incubator at 37°C for 2 hours. After discarding the liquid, the plates were blocked with 200µl/well of blocking solution containing 5% skim milk (Guangming skim milk) in PBS, and incubated in the incubator at 37°C for 2.5 hours or overnight at 4°C (16-18 hours). After blocking, the blocking solution was discarded and the plate was washed 5 times with PBST buffer (PBS containing 0.05% tweeen-20, pH7.4). Cynomolgus monkey thrombin protein with His tag (SEQ ID NO: 3, as shown by the double underline) was diluted with sample dilution (PBS containing 1%BSA, PH7.4) to 0.5µg/ml and was added to each well, 50µl/well. Then the plate was incubated in the incubator at 37°C for 2h or overnight. After incubation, the blocking solution in the plate was discarded, and the plate was washed with PBST for 6 times, and then was added with 50µl of various concentrations of the test antibodies diluted with sample dilution and the plate was incubated at 37°C for 2h. The plates was washed 5 times with PBST after incubation, and was added with 100µl/well of goat anti-human secondary antibody (Jackson Immuno Research,Cat No. 109-035-003) labeled with HRP, diluted in sample dilution, and the plate was incubated at 37°C for 1h. After washing the plates 5 times with PBST, 50µl/well of TMB chromogenic substrate (KPL, Cat No. 52-00-03) was added to each well, and incubated at room temperature for 10-15 min, the reaction was stopped by the addition of 50µl/well 1M H₂SO₄ to each well. The OD value at a wavelength of 450nm was read on NOVOStar microplate reader, and then EC50 values of the binding of thrombin antibodies to cynomolgus monkey thrombin were calculated. The results are shown in Table 8.

**Table 8. The cross binding activity of the antibodies in present invention with different species thrombin.**

| Antibody of the invention | Monkey thrombin EC50(nM) |
|---|---|
| H1601-008 | 11.18 |

The data showed that the antibody in the present invention showed excellent binding activities to monkey thrombin.

### Test 4. BIAcore assay for the affinity of the thrombin antibodies

The anti-human capture antibody was covalently linked to the CM5 biochip (Cat. # BR-1000-12, GE) according to the method described in the anti-human trapping kit (Cat. # BR-1008-39, GE), for affinity capture of the test antibodies. Then, the thrombin antibodies and human thrombin with His tag were flowed through the surface of the biochip, and the reaction signal was detected in real time using a Biacore instrument to obtain the binding and dissociation curves, the value of affinity was obtained by fitting. After each cycle of dissociation is finished in the experiment, the biochip was washed and regenerated with a regeneration solution provided in the anti-human capture kit. The results are shown in table 9.

**Table 9. Affinity activities of the antibodies in the present invention tohuman thrombin**

| **Antibody of the invention** | **Human thrombin BIAcoreKD (nM)** | **Human Prothrombin BIAcoreKD (nM)** |
|---|---|---|
| H1601-008 | 8.97 | 94.3 |
| H1601-009 | 8.17 | -- |

The results demonstrate that the antibodies h1601-008 and h1601-009 in present invention have the same excellent binding activity and affinity to human thrombin as CH-160.

Affinity tests were performed on the antibodies screened by the phage display technique (Example 6). The results obtained are shown in Table 10.

**Table 10. Test results of antibody Biacore test**

| **Sample** | **KD(M)** | | **Fold of Affinity relative to h1601-008** | | **Selective activity** |
|---|---|---|---|---|---|
| | **Thrombin** | **Pro-Thrombin** | **Thrombin** | **Pro- Thrombin** | |
| Ichorcumab | 2.57E-09 | 1.31E-07 | 3.0 | 0.6 | 51.0 |
| h1601-008 | 7.78E-09 | 7.68E-08 | 1.0 | 1.0 | 9.9 |
| aTM-1 | 1.91E-10 | 3.48E-09 | 40.7 | 22.1 | 18.2 |
| aTM-2 | 8.99E-11 | 1.23E-09 | 86.5 | 62.4 | 13.7 |
| aTM-4 | 1.44E-10 | 2.62E-09 | 54.0 | 29.3 | 18.2 |
| aTM-7 | 1.72E-10 | 2.05E-09 | 45.2 | 37.5 | 11.9 |

It can be seen from the above that the novel antibodies screened by phage display technology have greatly improved binding activity to thrombin, and the selectivity has also increased slightly.

### Test 5. The effects of thrombin antibodies on thrombinease activity

In this experiment, the effect of the thrombin antiboy on the activity of thrombinase was tested by measuring the enzymatic activity of thrombin on its substrate S2228.

Human thrombin with His tag was gradient diluted with PBS buffer, pH 7.4 to a concentration of 100nM, and added to a 96-well plate with black wall and transparent bottom, at a volume of 25µl/well. The test thrombin antibody of the present invention was gradient diluted with PBS to concentrations from 2000nM to 62.5nM (1:2 gradient dilution), and added to the plate at a volume of 25µl/well. After incubation at room temperature for 30-60 minute, S2228 (Substrate for the detection of thrombin activity, synthesized by Gill Biochemistry (Shanghai) Co., Ltd.) was diluted with PBS to a concentration of 4mM, 50µl/well was added to the plate of the previous step. For the negative control, added with thrombin or S2228 only. The OD value at a wavelength of 450nm was read on NOVOStar microplate reader after incubated at room temperature for 30 minutes. The results are shown in figure 2, wherein thrombin and S2228 indicate the OD value measured in the negative control well, 0.625:1, 1.25:1, 2.5:1, 5:1, 10:1, 20:1 indicate the OD value measured at different molar ratios of the test antibody and thrombin.

The results in Figure 2 show that binding of h1601-008 to thrombin does not affect the enzymatic activity on substrate S2228.

### Pharmacodynamic experiment of thrombin antibody

### Test 6. Normal human plasma APTT test

In this experiment, the effect of the antibody of the present invention on the APTT (activated partial thromboplastin time) value was tested by co-incubating normal human plasma with IgG and different concentrations of thrombin antibody.

IgG is a negative control, which is a human immunoglobulin obtained by using a conventional affinity chromatography method such as Protein A Purification; and h1601-008 is a test antibody with different concentrations according to the present invention.

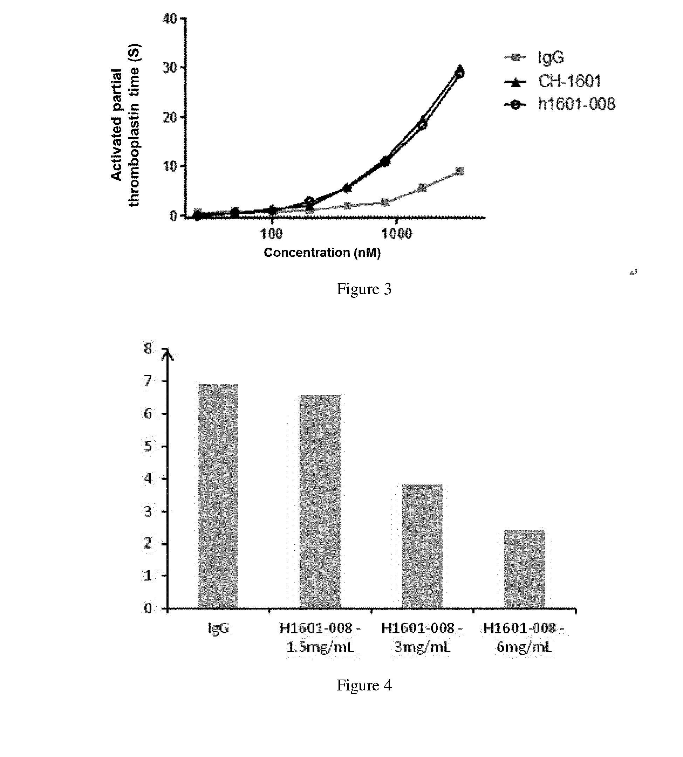

The results are shown in Figure 3. The data showed that with the increase of the concentrations of antibodies CH-1601 and h1601-008, the APTT value of normal human plasma was also increased; at the highest concentration of 3200 nM, h1601-008 increased the APTT value to a peak of 28.7 seconds (Table 11).

**Table 11. Increased APTT values in normal human plasma at different concentrations of the thrombin antibodies**

| **Antibody Concentration (nM)** | **25** | **50** | **100** | **200** | **400** | **800** | **1600** | **3200** |
|---|---|---|---|---|---|---|---|---|
| **IgG ΔAPTT (sec)** | 0.5 | 0.9 | 0.7 | 1.1 | 1.9 | 2.7 | 5.6 | 8.9 |
| **CH-1601 ΔAPTT (sec)** | 0.3 | 0.5 | 1.3 | 2.0 | 5.8 | 11.4 | 19.6 | 29.9 |
| **h1601-008 ΔAPTT (sec)** | 0.0 | 0.6 | 0.9 | 2.9 | 5.5 | 10.9 | 18.2 | 28.7 |

### Test 7. Derivative circulation model of cynomolgus monkey

16 healthy cynomolgus monkeys (Half male and half female, provided by Hainan Jingang biotechnology Limited by Share Ltd) were adapted to experimental environment for two weeks and weighed. They were divided into 4 groups and each group had 4 monkeys according to weight. The animals were anesthetized with Zoletil, then further anesthetized with pentobarbital. Before operation, the different groups were slowly injected via lower extremity vein with the antibodies of the present invention and the control drug (see Table 12 for the drug and dosage). The following steps were performed on the thermostatic operating table at 37°C. The femoral artery and vein were isolated surgically under the groin, in the order from the left side to the right side. Arteriovenous catheterization was performed by surgery. After the cannula was fixed, the artery clamp was released (the artery clamp on the left side was released 15 minutes after administration, and on the right side was released 25 minutes after administration) and counting at the same time, and the blood flow lasted for 15 minutes. The two ends of the cannula were clamped with hemostats or vascular clamps to prevent emboli shedding. The silicone tube was removed and placed into a petri dish containing physiological saline. Then procoagulant line with the thrombus was removed by usiung a surgical scissors. Both ends were placed on paper towel to make water absorbed for 3 seconds, then weighed with precision electronic scale and the net weight of the thrombus was calculated.

**Table 12**

| Group | Animal # | Drug | Dosage | Volume of administration | Thrombus weight(mg) |
|---|---|---|---|---|---|
| 1 | 4 | IgG | 6 mg/kg | 5 mL/kg | 6.88 |
| 2 | 4 | H1601-008 | 1.5 mg/kg | 5 mL/kg | 6.56 |
| 3 | 4 | H1601-008 | 3 mg/kg | 5 mL/kg | 3.83 |
| 4 | 4 | H1601-008 | 6 mg/kg | 5 mL/kg | 2.40 |

The experimental results are shown in figure 4, H1601-008 significantly inhibited the formation of thrombosis in cynomolgus monkeys at doses of 3 mg/kg and 6 mg/kg. There was a statistically significant difference compared to the average thrombus weight of the negative antibody group (IgG-6 mg/kg).

The statistical methods are One-way ANOVA and Student t test. Compared with group IgG, *p < 0.05, **p < 0.01, ***p < 0.001.

### Test 8. Determination for CT2 value and estimation of treatment window

In this experiment, the effect of the antibodies of the present invention on the APTT value was tested by co-incubating normal cynomolgus monkey plasma with different concentrations of thrombin antibody (refer to test 6).

The results of experiment are shown in Figure 5. With the increase of the concentration of antibody H1601-008, the APTT value of normal monkey plasma was prolonged obviously. APTT value reached a peak of 66.9 seconds when H1601-008 was at the highest concentration of 25600 nM (table 13).

**Table 13. Effects of different concentrations of thrombin antibody on APTT in normal cynomolgus monkey plasma**

| Antibody Concentration (nM) | **0** | **200** | **400** | **800** | **1600** | **3200** | **6400** | **12800** | **25600** |
|---|---|---|---|---|---|---|---|---|---|
| **IgG APTT (sec)** | 18.5 | 19.0 | 19.0 | 18.6 | 18.7 | 19.0 | 19.3 | 19.4 | 19.9 |
| **H1601-008 APTT (sec)** | 19.0 | 19.1 | 20.1 | 23.9 | 28.9 | 35.9 | 45.1 | 56.8 | 66.9 |

### Test 9. Pharmacokinetic test of the thrombin antibody of the present invention

Using cynomolgus monkeys as test animals, serum concentrations in serum of cynomolgus monkeys after administration were measured by ELISA (see Test4), and the t1/2 and main parameters of the drugs to be tested were calculated by Winnolin software and EXCEL. The pharmacokinetic characteristics of the compounds of the present invention in cynomolgus monkeys were studied and their pharmacokinetic characteristics were evaluated

Experimental cynomolgus monkeys were common, male, 3-5 years old, 3.5 - 4.5 kilograms, and feeding by Chengdu Huaxi Hai Medicine Technology Co., Ltd. Feeding environment: ordinary room. The cynomolgus monkeys were adapted to experimental environment for not less than 3 days, with 12/12-hour light/dark cycle, at temperature of 16-26°C, and with relative humidity of 40-60%. The cynomolgus monkeys were grouped, each group of 3 animals. On the day of experiment, each cynomolgus monkey was intravenously injected with test drug with a dose of 3 mg/kg and 10 mg/kg, respectively. The volume of intravenous injection was 10 ml/kg, and the speed of administration was 2-4ml/minute.

The time point on which blood was taken was 0.25h, 2 h, 4 h, 8 h, 1 d, 2 d, 3 d, 4 d, 7 d, 10 d, 14 d, 21 d, 28 d, 35 d after administration. About 1 mL of blood was taken each time, heparin sodium was used as an anticoagulant, the samples were temporarily stored in ice, and centrifuged at 1800g at 2-8°C for 10 minutes, and stored at -70°C.

Serum concentrations in serum of cynomolgus monkeys were measured by ELISA, and the t1/2 and main parameters of the drugs to be tested were calculated by Winnolin software and EXCEL, the results are shown as follows.

Pharmacokinetic parameters of the antibody of the present invention are shown in the following table 14,

**Table 14**

| Example | Dosage | Cmax(ug/ml) | AUC 0-∞ (ug/ml*h) | CLz/F(ml/min/kg) | Vz/F(ml/kg) | t1/2(h) |
|---|---|---|---|---|---|---|
| H1601-008 | 3 mg/kg | 52.74±9.59 | 9024±1086 | 0.0056±0.0007 | 69.86±10.34 | 151±23 |
| | 10 mg/kg | 98.81±3.3 | 20054±5992 | 0.009±0.003 | 143±43 | 189±41 |

Conclusion: The pharmacokinetic properties of the antibody of the present invention are good

## Claims

1. A thrombin antibody or antigen-binding fragment thereof, comprising one or more CDR region selected from the following sequence or an amino acid sequence having at least 95% identity to the following sequences:
HCDR region of antibody heavy chain: SEQ ID NO: 7, SEQ ID NO:8 and SEQ ID NO: 21; and
LCDR region of antibody light chain: SEQ ID NO:10, SEQ ID NO:11 and SEQ ID NO: 12;
wherein the sequence of SEQ ID NO: 21 is represented by formula (I):
DHYX₁G X₂SYVFDX₃ (I);
wherein X₁ is selected from the group consisting of H, I, L and M; X₂ is selected from N and A; X₃ is selected from the group consisting of Y, S, L and T.

2. The thrombin antibody or the antigen-binding fragment thereof according to claim 1, wherein the thrombin antibody or the antigen-binding fragment thereof comprises a HCDR1, a HCDR2, and a HCDR3 as shown in SEQ ID NO:7, SEQ ID NO:8, and SEQ ID NO: 21, respectively, or an amino acid sequence having at least 95% identity to SEQ ID NO:7, SEQ ID NO:8, and SEQ ID NO: 21.

3. The thrombin antibody or the antigen-binding fragment thereof according to claim 1, wherein the sequence of formula (I) is selected from the following sequence:
DHYHGNSYVFDY SEQ ID NO: 9;
DHYIGASYVFDY SEQ ID NO: 23;
DHYLGNSYVFDS SEQ ID NO: 24;
DHYLGNSYVFDL SEQ ID NO: 25; or
DHYMGNSYVFDT SEQ ID NO: 26.

4. The thrombin antibody or the antigen-binding fragment thereof according to claim 1, wherein the thrombin antibody or the antigen-binding fragment thereof comprises a LCDR1, a LCDR2, and a LCDR3 as shown in SEQ ID NO: 10, SEQ ID NO: 11, and SEQ ID NO: 12, respectively, or an amino acid sequence having at least 95% identity to SEQ ID NO: 10, SEQ ID NO: 11, and SEQ ID NO: 12.

5. The thrombin antibody or the antigen-binding fragment thereof according to claim 1, wherein the thrombin antibody or the antigen-binding fragment thereof is a murine antibody or the functional fragment thereof.

6. The thrombin antibody or the antigen-binding fragment thereof according to any one of claims 1-5, wherein the thrombin antibody or the antigen-binding fragment thereof further comprises a light chain FR region derived from murine κ chain or a variant thereof, or further comprises a light chain FR region derived from murine λ chain or a variant thereof; and/or wherein the thrombin antibody or the antigen-binding fragment thereof further comprises a heavy chain FR region derived from murine IgG1 or a variant thereof, or further comprises a heavy chain FR region derived from murine IgG2 or a variant thereof, or further comprises a heavy chain FR region derived from murine IgG3 or a variant thereof.

7. The thrombin antibody or the antigen-binding fragment thereof according to any one of claims 1-6, wherein the thrombin antibody or the antigen-binding fragment thereof comprises a heavy chain variable region of SEQ ID NO: 5 and a light chain variable region of SEQ ID NO: 6.

8. The thrombin antibody or the antigen-binding fragments thereof according to any one of claims 1-7, wherein the thrombin antibody or the antigen-binding fragment thereof further comprises a light chain constant region derived from murine κ chain or a variant thereof, or a light chain constant region derived from murine λ chain or a variant thereof; and/or wherein the thrombin antibody or the antigen-binding fragment thereof further comprises a heavy chain constant region derived from murine IgG1 or a variant thereof, or a heavy chain constant region derived from murine IgG2 or a variant thereof, or a heavy chain constant region derived from murine IgG3 or a variant thereof.

9. The thrombin antibody or the antigen-binding fragment thereof according to claim 1, wherein the thrombin antibody or the antigen-binding fragment thereof is a chimeric antibody or the functional fragment thereof.

10. The thrombin antibody or the antigen-binding fragment thereof according to claim 1, wherein the thrombin antibody or the antigen-binding fragment thereof is a humanized antibody or the functional fragment thereof.

11. The thrombin antibody or the antigen-binding fragment thereof according to claim 10, wherein the heavy chain FR region of the humanized antibody or the functional fragment thereof is derived from a combination sequence of human germline heavy chains IGHV3-23*04 and hjh6.1 or a mutant sequence thereof; wherein the humanized antibody or the functional fragment thereof comprises FR1, FR2, FR3 region from human germline heavy chain IGHV3-23*04 and FR4 region from human germline heavy chain hjh6.1, or a mutant sequence thereof.

12. The thrombin antibody or the antigen-binding fragment thereof according to claim 11, wherein the humanized antibody or the functional fragment thereof comprises a heavy chain variable region of SEQ ID NO: 13, or an amino acid sequence having at least 95% identity to SEQ ID NO: 13; preferably an amino acid sequence having 1-10 amino acid changes compared to SEQ ID NO: 13.

13. The thrombin antibody or the antigen-binding fragment thereof according to claim 11, wherein the humanized antibody or the functional fragment thereof comprises a heavy chain FR region with 1-10 amino acid back-mutations, wherein the back-mutation is preferably selected from the group consisting of R87K, K98R, Q3K and S49A amino acid back-mutation.

14. The thrombin antibody or the antigen-binding fragment thereof according to any one of claims 11-13, wherein the humanized antibody or the functional fragment thereof comprises a heavy chain variable region of SEQ ID NO: 22 or SEQ ID NO: 15, or an amino acid sequence having at least 95% identity to SEQ ID NO: 22 or SEQ ID NO: 15;
wherein the sequence of SEQ ID NO: 22 is represented by formula (II): 
EVQLVESGGGLVQPGGSLRLSCAASGFTFSDYYMAWVRQAPGKGLEWVSNI
NSDGSSTYYLDSLKSRFTISRDNSKNTLYLQMNSLKAEDTAVYYCARDHYX1G
X2SYVFDX3WGQGTTVTVSS (II);
 wherein X₁ is selected from the group consisting of H, I, L and M; X₂ is selected from N and A; X₃ is selected from the group consisting of Y, S, L and T.

15. The thrombin antibody or the antigen-binding fragments thereof according to claim 14, wherein the sequence of formula (II) is selected from the group consisting of SEQ ID NO: 14, SEQ ID NO: 27, SEQ ID NO: 28, SEQ ID NO: 29 and SEQ ID NO: 30.

16. The thrombin antibody or the antigen-binding fragment thereof according to claim 10, wherein the light chain FR region of the humanized antibody or the functional fragment thereof is derived from a combination sequence of human germline light chains IGKV1-39*01 and hjk4.1 or a mutant sequence thereof; wherein the humanized antibody or the functional fragment thereof comprises FR1, FR2, FR3 region from human germline light chain IGKV1-39*01 and FR4 region from hjk4.1 or a mutant sequence thereof.

17. The thrombin antibody or the antigen-binding fragment thereof according to claim 16, wherein the humanized antibody or the functional fragment thereof comprises a light chain variable region of SEQ ID NO: 16, or an amino acid sequence having at least 95% identity to SEQ ID NO: 16; preferably an amino acid sequence having 1-10 amino acid changes compared to SEQ ID NO: 16.

18. The thrombin antibody or the antigen-binding fragment thereof according to claim 16, wherein the humanized antibody or the functional fragment thereof comprises light chain FR region with 1-10 amino acid back-mutations, wherein the back-mutation is preferably selected from the group consisting of Y49S, T69K, K45R, L47I and F71Y amino acid back-mutation.

19. The thrombin antibody or the antigen-binding fragment thereof according to any one of claims 16-18, wherein the humanized antibody or the functional fragment thereof comprises a light chain variable region of SEQ ID NO: 17 or SEQ ID NO: 18, or an amino acid sequence having at least 95% identity to SEQ ID NO: 17 or SEQ ID NO: 18.

20. The thrombin antibody or the antigen-binding fragment thereof according to claim 10, wherein the humanized antibody or the functional fragment thereof comprises:
a̅) heavy chain variable region: wherein the sequence of the heavy chain variable region is selected from the group consisting of SEQ ID NO:13, SEQ ID NO:22, SEQ ID NO: 15, or an amino acid sequence having at least 95% identity to SEQ ID NO:13, SEQ ID NO:22, or SEQ ID NO: 15; and/or
b) light chain variable region: wherein the sequence of the light chain variable region is selected from the group consisting of SEQ ID NO:16, SEQ ID NO:17 and SEQ ID NO: 18, or an amino acid sequence having at least 95% identity to SEQ ID NO:16, SEQ ID NO:17 or SEQ ID NO: 18; wherein SEQID NO: 22 is as defined in claim 14.

21. The thrombin antibody or the antigen-binding fragment thereof according to any one of claims 9-20, wherein the thrombin antibody or the antigen-binding fragment further comprises a heavy chain constant region derived from human IgG1, IgG2, IgG3, IgG4 or a variant thereof, preferably comprises a heavy chain constant region derived from human IgG4, most preferably comprises a heavy chain constant region having the sequence of SEQ ID NO: 19.
wherein the thrombin antibody or the antigen-binding fragment further comprises a light chain constant region derived from human κ chain, human λ chain or a variant thereof, most preferably comprises a light chain constant region of SEQ ID NO: 20.

22. The thrombin antibody or the antigen-binding fragment thereof according to any one of claims 1-21, wherein the antigen-binding fragment is selected from the group consisting of Fab, Fab', F(ab')₂, single-chain antibody(scFv), dimerized V region (diabody), disulfide stabilized V region (dsFv) and antigen-binding fragment comprising peptide of CDR.

23. The thrombin antibody or the antigen-binding fragment thereof according to any one of claims 1-22, wherein the thrombin antibody or the antigen-binding fragment thereof binds to one or more residues selected from the group consisting of R70, Y71, E72, R73, N74, 175 and Y114 of thrombin; preferably, the thrombin antibody or the antigen-binding fragment thereof further binds to residues 9, 24, 60, 62, 64, 66, 69, 78 and 113 of thrombin.

24. An isolated monoclonal antibody or antigen-binding fragment thereof, that binds to one or more residues selected from the group consisting of R70, Y71, E72, R73, N74, 175, and Y114 of thrombin; preferably, it further binds to residues 9, 24, 60, 62, 64, 66, 69, 78 and 113 of thrombin.

25. A pharmaceutical composition, comprising therapeutically effective amount of the thrombin antibody or the antigen-binging fragment thereof according to any one of claims 1-24, and one or more pharmaceutically acceptable carriers, diluents or excipients.

26. A DNA molecule encoding the thrombin antibody or antigen-binding fragment thereof according to any one of claims 1-24.

27. An expression vector comprising the DNA molecule according to claim 26.

28. A host cell transformed with the expression vector according to claim 27, wherein the host cell is selected from the group consisting of prokaryotic cells and eukaryotic cells, preferably eukaryotic cells, more preferably mammalian cells.

29. The use of the thrombin antibody or antigen-binding fragment thereof according to any one of claims 1-24 or the pharmaceutical composition according to claim 25 or the DNA molecule according to claim 26, in the preparation of a medicament for treatment of thrombin-mediated disease or condition, wherein the disease or condition is preferably a thrombotic disease; more preferably venous thrombosis and pulmonary embolism, arterial thrombosis, thrombosis-induced stroke and peripheral arterial formation, atherosclerotic disease, cerebral arterial disease, or peripheral arterial disease; most preferably venous thrombosis, thrombosis-induced stroke, and atherosclerosis.

30. An agent for detecting or measuring human thrombin, wherein the agent comprises the antibody or antigen-binding fragment thereof according to any one of claims 1 to 24.


SEQUENCE LISTING

<110> Jiangsu hengrui medicine CO.,LTD Shanghai hengrui pharmaceutical
co.,LTD

<120> THROMBIN ANTIBODY, ANTIGEN-BINDING FRAGMENT AND PHARMACEUTICAL USE
THEREOF

<130> 2016

<160> 26

<170> PatentIn version 3.3

<210> 1
<211> 630
<212> PRT
<213> Artificial sequence

<220>
<223> h-prothrombin-Flag

<400> 1

Met Ala His Val Arg Gly Leu Gln Leu Pro Gly Cys Leu Ala Leu Ala
1               5                   10                  15

Ala Leu Cys Ser Leu Val His Ser Gln His Val Phe Leu Ala Pro Gln
            20                  25                  30

Gln Ala Arg Ser Leu Leu Gln Arg Val Arg Arg Ala Asn Thr Phe Leu
        35                  40                  45

Glu Glu Val Arg Lys Gly Asn Leu Glu Arg Glu Cys Val Glu Glu Thr
    50                  55                  60

Cys Ser Tyr Glu Glu Ala Phe Glu Ala Leu Glu Ser Ser Thr Ala Thr
65                  70                  75                  80

Asp Val Phe Trp Ala Lys Tyr Thr Ala Cys Glu Thr Ala Arg Thr Pro
                85                  90                  95

Arg Asp Lys Leu Ala Ala Cys Leu Glu Gly Asn Cys Ala Glu Gly Leu
            100                 105                 110

Gly Thr Asn Tyr Arg Gly His Val Asn Ile Thr Arg Ser Gly Ile Glu
        115                 120                 125

Cys Gln Leu Trp Arg Ser Arg Tyr Pro His Lys Pro Glu Ile Asn Ser
    130                 135                 140

Thr Thr His Pro Gly Ala Asp Leu Gln Glu Asn Phe Cys Arg Asn Pro
145                 150                 155                 160

Asp Ser Ser Thr Thr Gly Pro Trp Cys Tyr Thr Thr Asp Pro Thr Val
                165                 170                 175

Arg Arg Gln Glu Cys Ser Ile Pro Val Cys Gly Gln Asp Gln Val Thr
            180                 185                 190

Val Ala Met Thr Pro Arg Ser Glu Gly Ser Ser Val Asn Leu Ser Pro
        195                 200                 205

Pro Leu Glu Gln Cys Val Pro Asp Arg Gly Gln Gln Tyr Gln Gly Arg
    210                 215                 220

Leu Ala Val Thr Thr His Gly Leu Pro Cys Leu Ala Trp Ala Ser Ala
225                 230                 235                 240

Gln Ala Lys Ala Leu Ser Lys His Gln Asp Phe Asn Ser Ala Val Gln
                245                 250                 255

Leu Val Glu Asn Phe Cys Arg Asn Pro Asp Gly Asp Glu Glu Gly Val
            260                 265                 270

Trp Cys Tyr Val Ala Gly Lys Pro Gly Asp Phe Gly Tyr Cys Asp Leu
        275                 280                 285

Asn Tyr Cys Glu Glu Ala Val Glu Glu Glu Thr Gly Asp Gly Leu Asp
    290                 295                 300

Glu Asp Ser Asp Arg Ala Ile Glu Gly Arg Thr Ala Thr Ser Glu Tyr
305                 310                 315                 320

Gln Thr Phe Phe Asn Pro Arg Thr Phe Gly Ser Gly Glu Ala Asp Cys
                325                 330                 335

Gly Leu Arg Pro Leu Phe Glu Lys Lys Ser Leu Glu Asp Lys Thr Glu
            340                 345                 350

Arg Glu Leu Leu Glu Ser Tyr Ile Asp Gly Arg Ile Val Glu Gly Ser
        355                 360                 365

Asp Ala Glu Ile Gly Met Ser Pro Trp Gln Val Met Leu Phe Arg Lys
    370                 375                 380

Ser Pro Gln Glu Leu Leu Cys Gly Ala Ser Leu Ile Ser Asp Arg Trp
385                 390                 395                 400

Val Leu Thr Ala Ala His Cys Leu Leu Tyr Pro Pro Trp Asp Lys Asn
                405                 410                 415

Phe Thr Glu Asn Asp Leu Leu Val Arg Ile Gly Lys His Ser Arg Thr
            420                 425                 430

Arg Tyr Glu Arg Asn Ile Glu Lys Ile Ser Met Leu Glu Lys Ile Tyr
        435                 440                 445

Ile His Pro Arg Tyr Asn Trp Arg Glu Asn Leu Asp Arg Asp Ile Ala
    450                 455                 460

Leu Met Lys Leu Lys Lys Pro Val Ala Phe Ser Asp Tyr Ile His Pro
465                 470                 475                 480

Val Cys Leu Pro Asp Arg Glu Thr Ala Ala Ser Leu Leu Gln Ala Gly
                485                 490                 495

Tyr Lys Gly Arg Val Thr Gly Trp Gly Asn Leu Lys Glu Thr Trp Thr
            500                 505                 510

Ala Asn Val Gly Lys Gly Gln Pro Ser Val Leu Gln Val Val Asn Leu
        515                 520                 525

Pro Ile Val Glu Arg Pro Val Cys Lys Asp Ser Thr Arg Ile Arg Ile
    530                 535                 540

Thr Asp Asn Met Phe Cys Ala Gly Tyr Lys Pro Asp Glu Gly Lys Arg
545                 550                 555                 560

Gly Asp Ala Cys Glu Gly Asp Ser Gly Gly Pro Phe Val Met Lys Ser
                565                 570                 575

Pro Phe Asn Asn Arg Trp Tyr Gln Met Gly Ile Val Ser Trp Gly Glu
            580                 585                 590

Gly Cys Asp Arg Asp Gly Lys Tyr Gly Phe Tyr Thr His Val Phe Arg
        595                 600                 605

Leu Lys Lys Trp Ile Gln Lys Val Ile Asp Gln Phe Gly Glu Asp Tyr
    610                 615                 620

Lys Asp Asp Asp Asp Lys
625                 630

<210>  2
<211>  628
<212>  PRT
<213>  Artificial sequence

<220>
<223>  h-prothrombin-His

<400>  2

Met Ala His Val Arg Gly Leu Gln Leu Pro Gly Cys Leu Ala Leu Ala
1               5                   10                  15

Ala Leu Cys Ser Leu Val His Ser Gln His Val Phe Leu Ala Pro Gln
            20                  25                  30

Gln Ala Arg Ser Leu Leu Gln Arg Val Arg Arg Ala Asn Thr Phe Leu
        35                  40                  45

Glu Glu Val Arg Lys Gly Asn Leu Glu Arg Glu Cys Val Glu Glu Thr
    50                  55                  60

Cys Ser Tyr Glu Glu Ala Phe Glu Ala Leu Glu Ser Ser Thr Ala Thr
65                  70                  75                  80

Asp Val Phe Trp Ala Lys Tyr Thr Ala Cys Glu Thr Ala Arg Thr Pro
                85                  90                  95

Arg Asp Lys Leu Ala Ala Cys Leu Glu Gly Asn Cys Ala Glu Gly Leu
            100                 105                 110

Gly Thr Asn Tyr Arg Gly His Val Asn Ile Thr Arg Ser Gly Ile Glu
        115                 120                 125

Cys Gln Leu Trp Arg Ser Arg Tyr Pro His Lys Pro Glu Ile Asn Ser
    130                 135                 140

Thr Thr His Pro Gly Ala Asp Leu Gln Glu Asn Phe Cys Arg Asn Pro
145                 150                 155                 160

Asp Ser Ser Thr Thr Gly Pro Trp Cys Tyr Thr Thr Asp Pro Thr Val
                165                 170                 175

Arg Arg Gln Glu Cys Ser Ile Pro Val Cys Gly Gln Asp Gln Val Thr
            180                 185                 190

Val Ala Met Thr Pro Arg Ser Glu Gly Ser Ser Val Asn Leu Ser Pro
        195                 200                 205

Pro Leu Glu Gln Cys Val Pro Asp Arg Gly Gln Gln Tyr Gln Gly Arg
    210                 215                 220

Leu Ala Val Thr Thr His Gly Leu Pro Cys Leu Ala Trp Ala Ser Ala
225                 230                 235                 240

Gln Ala Lys Ala Leu Ser Lys His Gln Asp Phe Asn Ser Ala Val Gln
                245                 250                 255

Leu Val Glu Asn Phe Cys Arg Asn Pro Asp Gly Asp Glu Glu Gly Val
            260                 265                 270

Trp Cys Tyr Val Ala Gly Lys Pro Gly Asp Phe Gly Tyr Cys Asp Leu
        275                 280                 285

Asn Tyr Cys Glu Glu Ala Val Glu Glu Glu Thr Gly Asp Gly Leu Asp
    290                 295                 300

Glu Asp Ser Asp Arg Ala Ile Glu Gly Arg Thr Ala Thr Ser Glu Tyr
305                 310                 315                 320

Gln Thr Phe Phe Asn Pro Arg Thr Phe Gly Ser Gly Glu Ala Asp Cys
                325                 330                 335

Gly Leu Arg Pro Leu Phe Glu Lys Lys Ser Leu Glu Asp Lys Thr Glu
            340                 345                 350

Arg Glu Leu Leu Glu Ser Tyr Ile Asp Gly Arg Ile Val Glu Gly Ser
        355                 360                 365

Asp Ala Glu Ile Gly Met Ser Pro Trp Gln Val Met Leu Phe Arg Lys
    370                 375                 380

Ser Pro Gln Glu Leu Leu Cys Gly Ala Ser Leu Ile Ser Asp Arg Trp
385                 390                 395                 400

Val Leu Thr Ala Ala His Cys Leu Leu Tyr Pro Pro Trp Asp Lys Asn
                405                 410                 415

Phe Thr Glu Asn Asp Leu Leu Val Arg Ile Gly Lys His Ser Arg Thr
            420                 425                 430

Arg Tyr Glu Arg Asn Ile Glu Lys Ile Ser Met Leu Glu Lys Ile Tyr
        435                 440                 445

Ile His Pro Arg Tyr Asn Trp Arg Glu Asn Leu Asp Arg Asp Ile Ala
    450                 455                 460

Leu Met Lys Leu Lys Lys Pro Val Ala Phe Ser Asp Tyr Ile His Pro
465                 470                 475                 480

Val Cys Leu Pro Asp Arg Glu Thr Ala Ala Ser Leu Leu Gln Ala Gly

485                 490                 495

Tyr Lys Gly Arg Val Thr Gly Trp Gly Asn Leu Lys Glu Thr Trp Thr
            500                 505                 510

Ala Asn Val Gly Lys Gly Gln Pro Ser Val Leu Gln Val Val Asn Leu
        515                 520                 525

Pro Ile Val Glu Arg Pro Val Cys Lys Asp Ser Thr Arg Ile Arg Ile
    530                 535                 540

Thr Asp Asn Met Phe Cys Ala Gly Tyr Lys Pro Asp Glu Gly Lys Arg
545                 550                 555                 560

Gly Asp Ala Cys Glu Gly Asp Ser Gly Gly Pro Phe Val Met Lys Ser
                565                 570                 575

Pro Phe Asn Asn Arg Trp Tyr Gln Met Gly Ile Val Ser Trp Gly Glu
            580                 585                 590

Gly Cys Asp Arg Asp Gly Lys Tyr Gly Phe Tyr Thr His Val Phe Arg
        595                 600                 605

Leu Lys Lys Trp Ile Gln Lys Val Ile Asp Gln Phe Gly Glu His His
    610                 615                 620

His His His His
625

<210>  3
<211>  626
<212>  PRT
<213>  Artificial sequence

<220>
<223>  Cyno-prothrombin-His

<400>  3

Met Ala His Val Arg Gly Leu Gln Leu Pro Gly Cys Leu Ala Leu Ala
1               5                   10                  15

Ala Leu Cys Ser Leu Val His Ser Gln His Val Phe Leu Ala Pro Gln
            20                  25                  30

Gln Ala Leu Ser Leu Leu Gln Arg Val Arg Arg Ala Ser Ser Gly Phe
        35                  40                  45

Leu Glu Glu Val Phe Lys Gly Asn Leu Glu Arg Glu Cys Val Glu Glu
    50                  55                  60

Thr Cys Ser Tyr Glu Glu Ala Phe Glu Ala Leu Glu Ser Ser Thr Ala
65                  70                  75                  80

Thr Asp Ala Phe Trp Ala Lys Tyr Thr Ala Cys Glu Thr Ala Arg Thr
                85                  90                  95

Ser Arg Asp Thr Leu Ala Ala Cys Leu Glu Gly Asn Cys Ala Glu Asp
            100                 105                 110

Leu Gly Thr Asn Tyr Arg Gly His Val Asn Ile Thr Arg Ser Gly Ile
        115                 120                 125

Glu Cys Gln Leu Trp Arg Ser Arg Tyr Pro His Lys Pro Glu Ile Asn
    130                 135                 140

Ser Thr Thr His Pro Gly Ala Asp Leu Gln Glu Asn Phe Cys Arg Asn
145                 150                 155                 160

Pro Asp Ser Ser Thr Thr Gly Pro Trp Cys Tyr Thr Thr Asp Pro Thr
                165                 170                 175

Val Arg Arg Glu Glu Cys Ser Ile Pro Val Cys Gly Gln Asp Gln Val
            180                 185                 190

Thr Val Val Met Thr Pro Arg Ser Ser Val Asn Leu Ser Leu Pro Ser
        195                 200                 205

Glu Glu Cys Val Pro Asp Arg Gly Arg Gln Tyr Gln Gly His Leu Ala
    210                 215                 220

Val Thr Thr His Gly Leu Pro Cys Leu Ala Trp Ala Ser Ala Gln Ala
225                 230                 235                 240

Lys Ala Leu Ser Lys His Gln Asp Phe Asp Ser Ala Val Gln Leu Val
                245                 250                 255

Glu Asn Phe Cys Arg Asn Pro Asp Gly Asp Glu Glu Gly Val Trp Cys
            260                 265                 270

Tyr Val Ala Gly Lys Pro Gly Asp Phe Glu Tyr Cys Asp Leu Asn Tyr
        275                 280                 285

Cys Glu Glu Ala Val Asp Glu Glu Thr Gly Asp Gly Leu Gly Glu Asp
    290                 295                 300

Pro Asp Arg Ala Ile Glu Gly Arg Thr Ala Thr Ser Glu Tyr Gln Thr

305                 310                 315                 320

Phe Phe Asp Pro Arg Thr Phe Gly Leu Gly Glu Ala Asp Cys Gly Leu
                325                 330                 335

Arg Pro Leu Phe Glu Lys Lys Ser Leu Glu Asp Lys Thr Glu Gly Glu
            340                 345                 350

Leu Leu Glu Ser Tyr Ile Asp Gly Arg Ile Val Glu Gly Trp Asp Ala
        355                 360                 365

Glu Ile Gly Met Ser Pro Trp Gln Val Met Leu Phe Arg Lys Ser Pro
    370                 375                 380

Gln Glu Leu Leu Cys Gly Ala Ser Leu Ile Ser Asp Arg Trp Val Leu
385                 390                 395                 400

Thr Ala Ala His Cys Leu Leu Tyr Pro Pro Trp Asp Lys Asn Phe Thr
                405                 410                 415

Glu Asn Asp Leu Leu Val Arg Ile Gly Lys His Ser Arg Thr Arg Tyr
            420                 425                 430

Glu Arg Asn Ile Glu Lys Ile Ser Met Leu Glu Lys Ile Tyr Ile His
        435                 440                 445

Pro Arg Tyr Asn Trp Arg Glu Asn Leu Asp Arg Asp Ile Ala Leu Met
    450                 455                 460

Lys Leu Lys Lys Pro Ile Thr Phe Ser Asp Tyr Ile His Pro Val Cys
465                 470                 475                 480

Leu Pro Asp Arg Glu Thr Ala Ala Ser Leu Phe Gln Ala Gly Tyr Lys
                485                 490                 495

Gly Arg Val Thr Gly Trp Gly Asn Leu Lys Glu Thr Trp Thr Thr Asn
            500                 505                 510

Val Gly Lys Val Gln Pro Ser Val Leu Gln Val Val Asn Leu Pro Ile
        515                 520                 525

Val Glu Arg Ser Val Cys Lys Asp Ser Thr Arg Ile Arg Ile Thr Asp
    530                 535                 540

Asn Met Phe Cys Ala Gly Tyr Lys Pro Gly Glu Gly Lys Arg Gly Asp
545                 550                 555                 560

Ala Cys Glu Gly Asp Ser Gly Gly Pro Phe Val Met Lys Asn Pro Leu
                565                 570                 575

Asn Lys Arg Trp Tyr Gln Met Gly Ile Val Ser Trp Gly Glu Gly Cys
            580                 585                 590

Asp Arg Asp Gly Lys Tyr Gly Phe Tyr Thr His Val Phe Arg Leu Lys
        595                 600                 605

Lys Trp Ile Gln Lys Val Ile Asp Gln Phe Gly Asp His His His His
    610                 615                 620

His His
625

<210>  4
<211>  624
<212>  PRT
<213>  Artificial sequence
<220>
<223>  m-prothrombin-His

<400>  4

Met Ser His Val Arg Gly Leu Gly Leu Pro Gly Cys Leu Ala Leu Ala
1               5                   10                  15

Ala Leu Val Ser Leu Val His Ser Gln His Val Phe Leu Ala Pro Gln
            20                  25                  30

Gln Ala Leu Ser Leu Leu Gln Arg Val Arg Arg Ala Asn Ser Gly Phe
        35                  40                  45

Leu Glu Glu Leu Arg Lys Gly Asn Leu Glu Arg Glu Cys Val Glu Glu
    50                  55                  60

Gln Cys Ser Tyr Glu Glu Ala Phe Glu Ala Leu Glu Ser Pro Gln Asp
65                  70                  75                  80

Thr Asp Val Phe Trp Ala Lys Tyr Thr Val Cys Asp Ser Val Arg Lys
                85                  90                  95

Pro Arg Glu Thr Phe Met Asp Cys Leu Glu Gly Arg Cys Ala Met Asp
            100                 105                 110

Leu Gly Val Asn Tyr Leu Gly Thr Val Asn Val Thr His Thr Gly Ile
        115                 120                 125

Gln Cys Gln Leu Trp Arg Ser Arg Tyr Pro His Lys Pro Glu Ile Asn
    130                 135                 140

Ser Thr Thr His Pro Gly Ala Asp Leu Lys Glu Asn Phe Cys Arg Asn
145                 150                 155                 160

Pro Asp Ser Ser Thr Thr Gly Pro Trp Cys Tyr Thr Thr Asp Pro Thr
                165                 170                 175

Val Arg Arg Glu Glu Cys Ser Val Pro Val Cys Gly Gln Glu Gly Arg
            180                 185                 190

Thr Thr Val Val Met Thr Pro Arg Ser Gly Gly Ser Lys Asp Asn Leu
        195                 200                 205

Ser Pro Pro Leu Gly Gln Cys Leu Thr Glu Arg Gly Arg Leu Tyr Gln
    210                 215                 220

Gly Asn Leu Ala Val Thr Thr Leu Gly Ser Pro Cys Leu Pro Trp Asn
225                 230                 235                 240

Ser Leu Pro Ala Lys Thr Leu Ser Lys Tyr Gln Asp Phe Asp Pro Glu
                245                 250                 255

Val Lys Leu Val Glu Asn Phe Cys Arg Asn Pro Asp Trp Asp Glu Glu
            260                 265                 270

Gly Ala Trp Cys Tyr Val Ala Gly Gln Pro Gly Asp Phe Glu Tyr Cys
        275                 280                 285

Asn Leu Asn Tyr Cys Glu Glu Ala Val Gly Glu Glu Asn Tyr Asp Val
    290                 295                 300

Asp Glu Ser Ile Ala Gly Arg Thr Thr Asp Ala Glu Phe His Thr Phe
305                 310                 315                 320

Phe Asn Glu Lys Thr Phe Gly Leu Gly Glu Ala Asp Cys Gly Leu Arg
                325                 330                 335

Pro Leu Phe Glu Lys Lys Ser Leu Lys Asp Thr Thr Glu Lys Glu Leu
            340                 345                 350

Leu Asp Ser Tyr Ile Asp Gly Arg Ile Val Glu Gly Trp Asp Ala Glu
        355                 360                 365

Lys Gly Ile Ala Pro Trp Gln Val Met Leu Phe Arg Lys Ser Pro Gln
    370                 375                 380

Glu Leu Leu Cys Gly Ala Ser Leu Ile Ser Asp Arg Trp Val Leu Thr

385                 390                 395                 400

Ala Ala His Cys Ile Leu Tyr Pro Pro Trp Asp Lys Asn Phe Thr Glu
                405                 410                 415

Asn Asp Leu Leu Val Arg Ile Gly Lys His Ser Arg Thr Arg Tyr Glu
            420                 425                 430

Arg Asn Val Glu Lys Ile Ser Met Leu Glu Lys Ile Tyr Val His Pro
        435                 440                 445

Arg Tyr Asn Trp Arg Glu Asn Leu Asp Arg Asp Ile Ala Leu Leu Lys
    450                 455                 460

Leu Lys Lys Pro Val Pro Phe Ser Asp Tyr Ile His Pro Val Cys Leu
465                 470                 475                 480

Pro Asp Lys Gln Thr Val Thr Ser Leu Leu Arg Ala Gly Tyr Lys Gly
                485                 490                 495

Arg Val Thr Gly Trp Gly Asn Leu Arg Glu Thr Trp Thr Thr Asn Ile
            500                 505                 510

Asn Glu Ile Gln Pro Ser Val Leu Gln Val Val Asn Leu Pro Ile Val
        515                 520                 525

Glu Arg Pro Val Cys Lys Ala Ser Thr Arg Ile Arg Ile Thr Asp Asn
    530                 535                 540

Met Phe Cys Ala Gly Phe Lys Val Asn Asp Thr Lys Arg Gly Asp Ala
545                 550                 555                 560

Cys Glu Gly Asp Ser Gly Gly Pro Phe Val Met Lys Ser Pro Phe Asn
                565                 570                 575

Asn Arg Trp Tyr Gln Met Gly Ile Val Ser Trp Gly Glu Gly Cys Asp
            580                 585                 590

Arg Lys Gly Lys Tyr Gly Phe Tyr Thr His Val Phe Arg Leu Lys Arg
        595                 600                 605

Trp Ile Gln Lys Val Ile Asp Gln Phe Gly His His His His His His
    610                 615                 620

<210>  5
<211>  121
<212>  PRT
<213>  Mus sp.

<400>  5

Glu Val Lys Leu Val Glu Ser Glu Gly Gly Leu Val Gln Pro Gly Ser
1               5                   10                  15

Ser Leu Lys Leu Ser Cys Thr Ala Ser Gly Phe Thr Phe Ser Asp Tyr
            20                  25                  30

Tyr Met Ala Trp Val Arg Gln Val Pro Glu Lys Gly Leu Glu Trp Val
        35                  40                  45

Ala Asn Ile Asn Ser Asp Gly Ser Ser Thr Tyr Tyr Leu Asp Ser Leu
    50                  55                  60

Lys Ser Arg Phe Ile Ile Ser Arg Asp Asn Ala Lys Asn Ile Leu Tyr
65                  70                  75                  80

Leu Gln Met Ser Ser Leu Lys Ser Glu Asp Thr Ala Thr Tyr Tyr Cys
                85                  90                  95

Ala Arg Asp His Tyr His Gly Asn Ser Tyr Val Phe Asp Tyr Trp Gly
            100                 105                 110

Gln Gly Thr Thr Leu Thr Val Ser Ser
        115                 120


<210>  6
<211>  107
<212>  PRT
<213>  Mus sp.

<400>  6

Asp Ile Gln Met Thr Gln Ser Ser Ser Ser Phe Ser Val Ser Arg Gly
1               5                   10                  15

Asn Arg Val Thr Ile Thr Cys Lys Ala Ser Glu Asp Ile Tyr Asn Arg
            20                  25                  30

Leu Ala Trp Tyr Gln Gln Lys Pro Gly Asn Ala Pro Arg Leu Ile Ile
        35                  40                  45

Ser Gly Ala Thr Ser Leu Glu Thr Gly Val Pro Ser Arg Phe Ser Gly
    50                  55                  60

Ser Gly Ser Gly Lys Asp Tyr Thr Leu Ser Ile Thr Ser Leu Gln Thr
65                  70                  75                  80

Glu Asp Val Gly Thr Tyr Tyr Cys Gln Gln Tyr Trp Ser Thr Pro Trp

85                  90                  95

Thr Phe Gly Gly Gly Thr Lys Leu Glu Ile Lys
            100                 105

<210>  7
<211>  5
<212>  PRT
<213>  Mus sp.

<400>  7

Asp Tyr Tyr Met Ala
1               5

<210>  8
<211>  17
<212>  PRT
<213>  Mus sp.

<400>  8

Asn Ile Asn Ser Asp Gly Ser Ser Thr Tyr Tyr Leu Asp Ser Leu Lys
1               5                   10                  15

Ser

<210>  9
<211>  12
<212>  PRT
<213>  Mus sp.

<400>  9

Asp His Tyr His Gly Asn Ser Tyr Val Phe Asp Tyr
1               5                   10

<210>  10
<211>  11
<212>  PRT
<213>  Mus sp.

<400>  10

Lys Ala Ser Glu Asp Ile Tyr Asn Arg Leu Ala
1               5                   10

<210>  11
<211>  7
<212>  PRT
<213>  Mus sp.

<400>  11

Gly Ala Thr Ser Leu Glu Thr

1               5


<210>  12
<211>  9
<212>  PRT
<213>  Mus sp.

<400>  12

Gln Gln Tyr Trp Ser Thr Pro Trp Thr
1               5


<210>  13
<211>  121
<212>  PRT
<213>  Artificial sequence

<220>
<223>   h1601-001 (CDR graft), humanized heavy chain variable region

<400>  13

Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Thr Phe Ser Asp Tyr
            20                  25                  30


Tyr Met Ala Trp Val Arg Gln Ala Pro Gly Lys Gly Leu Glu Trp Val
        35                  40                  45


Ser Asn Ile Asn Ser Asp Gly Ser Ser Thr Tyr Tyr Leu Asp Ser Leu
    50                  55                  60


Lys Ser Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Lys Asp His Tyr His Gly Asn Ser Tyr Val Phe Asp Tyr Trp Gly
            100                 105                 110


Gln Gly Thr Thr Val Thr Val Ser Ser
        115                 120


<210>  14
<211>  121
<212>  PRT
<213>  Artificial sequence

<220>
<223> humanized heavy chain variable region after back-mutation,

h1601-004/h1601-005/h1601-006

<400>  14

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Tyr Met Ala Trp Val Arg Gln Ala Pro Gly Lys Gly Leu Glu Trp Val
        35                  40                  45

Ser Asn Ile Asn Ser Asp Gly Ser Ser Thr Tyr Tyr Leu Asp Ser Leu
    50                  55                  60

Lys Ser Arg Phe Thr Ile Ser Arg Asp Asn Ser Lys Asn Thr Leu Tyr
65                  70                  75                  80

Leu Gln Met Asn Ser Leu Lys Ala Glu Asp Thr Ala Val Tyr Tyr Cys
                85                  90                  95

Ala Arg Asp His Tyr His Gly Asn Ser Tyr Val Phe Asp Tyr Trp Gly
            100                 105                 110

Gln Gly Thr Thr Val Thr Val Ser Ser
        115                 120


<210>  15
<211>  121
<212>  PRT
<213>  Artificial sequence

<220>
<223> humanized heavy chain variable region after back-mutation,
h1601-007/h1601-008/h1601-009

<400>  15

Glu Val Lys Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Tyr Met Ala Trp Val Arg Gln Ala Pro Gly Lys Gly Leu Glu Trp Val
        35                  40                  45

Ala Asn Ile Asn Ser Asp Gly Ser Ser Thr Tyr Tyr Leu Asp Ser Leu
    50                  55                  60

Lys Ser Arg Phe Thr Ile Ser Arg Asp Asn Ser Lys Asn Thr Leu Tyr
65                  70                  75                  80

Leu Gln Met Asn Ser Leu Lys Ala Glu Asp Thr Ala Val Tyr Tyr Cys
                85                  90                  95

Ala Arg Asp His Tyr His Gly Asn Ser Tyr Val Phe Asp Tyr Trp Gly
            100                 105                 110

Gln Gly Thr Thr Val Thr Val Ser Ser
        115                 120

<210>  16
<211>  107
<212>  PRT
<213>  Artificial sequence

<220>
<223>  humanized light chain variable region, h1601-001/h1601-004/h1601-007

<400>  16

Asp Ile Gln Met Thr Gln Ser Pro Ser Ser Leu Ser Ala Ser Val Gly
1               5                   10                  15

Asp Arg Val Thr Ile Thr Cys Lys Ala Ser Glu Asp Ile Tyr Asn Arg
            20                  25                  30

Leu Ala Trp Tyr Gln Gln Lys Pro Gly Lys Ala Pro Lys Leu Leu Ile
        35                  40                  45

Tyr Gly Ala Thr Ser Leu Glu Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Trp Ser Thr Pro Trp
                85                  90                  95

Thr Phe Gly Gly Gly Thr Lys Val Glu Ile Lys
            100                 105

<210>  17
<211>  107
<212>  PRT
<213>  Artificial sequence

<220>
<223> humanized light chain variable region after back-mutation,
h1601-002/h1601-005/h1601-008

<400>  17

Asp Ile Gln Met Thr Gln Ser Pro Ser Ser Leu Ser Ala Ser Val Gly
1               5                   10                  15

Asp Arg Val Thr Ile Thr Cys Lys Ala Ser Glu Asp Ile Tyr Asn Arg
            20                  25                  30

Leu Ala Trp Tyr Gln Gln Lys Pro Gly Lys Ala Pro Lys Leu Leu Ile
        35                  40                  45

Ser Gly Ala Thr Ser Leu Glu Thr Gly Val Pro Ser Arg Phe Ser Gly
    50                  55                  60

Ser Gly Ser Gly Lys Asp Phe Thr Leu Thr Ile Ser Ser Leu Gln Pro
65                  70                  75                  80

Glu Asp Phe Ala Thr Tyr Tyr Cys Gln Gln Tyr Trp Ser Thr Pro Trp
                85                  90                  95

Thr Phe Gly Gly Gly Thr Lys Val Glu Ile Lys
            100                 105


<210>  18
<211>  107
<212>  PRT
<213>  Artificial sequence

<220>
<223> humanized light chain variable region after back-mutation,
h1601-003/h1601-006/h1601-009

<400>  18

Asp Ile Gln Met Thr Gln Ser Pro Ser Ser Leu Ser Ala Ser Val Gly
1               5                   10                  15

Asp Arg Val Thr Ile Thr Cys Lys Ala Ser Glu Asp Ile Tyr Asn Arg
            20                  25                  30

Leu Ala Trp Tyr Gln Gln Lys Pro Gly Lys Ala Pro Arg Leu Ile Ile
        35                  40                  45

Ser Gly Ala Thr Ser Leu Glu Thr Gly Val Pro Ser Arg Phe Ser Gly
    50                  55                  60

Ser Gly Ser Gly Lys Asp Tyr Thr Leu Thr Ile Ser Ser Leu Gln Pro
65                  70                  75                  80

Glu Asp Phe Ala Thr Tyr Tyr Cys Gln Gln Tyr Trp Ser Thr Pro Trp
                85                  90                  95

Thr Phe Gly Gly Gly Thr Lys Val Glu Ile Lys
                100                 105

<210>  19
<211>  327
<212>  PRT
<213>  Artificial sequence

<220>
<223> heavy chain constant region, S228P mutation

<400>  19

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Pro Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325

<210>  20
<211>  107
<212>  PRT
<213>  Artificial sequence

<220>
<223>  light chain constant region of kappa

<400>  20

<210>  21
<211>  12
<212>  PRT
<213>  Artificial sequence

<220>
<223>  The formula sequence of HCDR3 obtained by phage display technology
screening

<220>
<221>  misc_feature
<222>  (4)..(4)
<223>  Xaa can be His, Ile,Leu or Met

<220>
<221>  misc_feature
<222>  (6)..(6)
<223>  Xaa can be Asn or Ala

<220>
<221>  misc_feature
<222>  (12)..(12)
<223>  Xaa can be Tyr,Ser,Leu or Thr

<400>  21

Asp His Tyr Xaa Gly Xaa Ser Tyr Val Phe Asp Xaa
1               5                   10

<210>  22
<211>  121
<212>  PRT
<213>  Artificial sequence

<220>
<223>  Heavy chian variable region obtained by phage display technology

<220>
<221>  misc_feature
<222>  (102)..(102)
<223>  Xaa can be His, Ile,Leu or Met

<220>
<221>  misc_feature
<222>  (104)..(104)
<223>  Xaa can be Asn or Ala

<220>
<221>  misc_feature

<222>  (110)..(110)
<223>  Xaa can be Tyr,Ser,Leu or Thr

<400>  22

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Tyr Met Ala Trp Val Arg Gln Ala Pro Gly Lys Gly Leu Glu Trp Val
        35                  40                  45

Ser Asn Ile Asn Ser Asp Gly Ser Ser Thr Tyr Tyr Leu Asp Ser Leu
    50                  55                  60

Lys Ser Arg Phe Thr Ile Ser Arg Asp Asn Ser Lys Asn Thr Leu Tyr
65                  70                  75                  80

Leu Gln Met Asn Ser Leu Lys Ala Glu Asp Thr Ala Val Tyr Tyr Cys
                85                  90                  95

Ala Arg Asp His Tyr Xaa Gly Xaa Ser Tyr Val Phe Asp Xaa Trp Gly
            100                 105                 110

Gln Gly Thr Thr Val Thr Val Ser Ser
        115                 120


<210>  23
<211>  12
<212>  PRT
<213>  Artificial sequence

<220>
<223>  aTM-1 heavy chain variable region HCDR3 sequence

<400>  23

Asp His Tyr Ile Gly Ala Ser Tyr Val Phe Asp Tyr
1               5                   10


<210>  24
<211>  12
<212>  PRT
<213>  Artificial sequence

<220>
<223>  aTM-2 heavy chain variable region HCDR3 sequence

<400>  24

Asp His Tyr Leu Gly Asn Ser Tyr Val Phe Asp Ser
1               5                   10

<210>  25
<211>  12
<212>  PRT
<213>  Artificial sequence

<220>
<223>  aTM-4 heavy chain variable region HCDR3 sequence

<400>  25

Asp His Tyr Leu Gly Asn Ser Tyr Val Phe Asp Leu
1               5                   10


<210>  26
<211>  12
<212>  PRT
<213>  Artificial sequence

<220>
<223>  aTM-7 heavy chain variable region HCDR3 sequence

<400>  26

Asp His Tyr Met Gly Asn Ser Tyr Val Phe Asp Thr
1               5                   10